# EUROPEAN PATENT SPECIFICATION

(11) **EP 4 023 759 B1**
(45) Date of publication and mention of the grant of the patent: **02.11.2022**
(21) Application number: 20217789.5
(22) Date of filing: 30.12.2020
(51) Int. Cl.: C12P 7/625, C12P 7/40, C12P 7/02, C12P 7/649, C12P 13/04, C12P 21/00

(54) **SYSTEMS FOR CO-CULTURE OF RALSTONIA EUTROPHA STRAINS**
SYSTEME FÜR CO-KULTUREN VON RALSTONIA EUTROPHA-STÄMMEN
SYSTÈMES DE CO-CULTURE DE SOUCHES DE RALSTONIA EUTROPHA

(43) Date of publication of application: 06.07.2022
(73) Proprietor: Bioextrax AB, 223 62 Lund (SE)
(72) Inventor: IBRAHIM, Mohammad H.A., 22647 Lund (SE)
(74) Representative: Høiberg P/S

(56) References cited:
- Bhatia S K: "Engineering of artificial microbial consortia of Ralstonia eutropha and Bacillus subtilis for poly(3-hydroxybutyrate-co-3-hydroxyvalerat e) copolymer production from sugarcane sugar without precursor feeding - PubMed", , 15 February 2018 (2018-02-15), pages 92-101, XP055809344, Retrieved from the Internet: URL:https://pubmed.ncbi.nlm.nih.gov/294864 11/ [retrieved on 2021-05-31]
- PRATAP R PATNAIK: "Cognitive optimization of microbial PHB production in an optimally dispersed bioreactor by single and mixed cultures", BIOPROCESS AND BIOSYSTEMS ENGINEERING, SPRINGER, BERLIN, DE, vol. 32, no. 4, 13 November 2008 (2008-11-13), pages 557-568, XP019716925, ISSN: 1615-7605
- MOHANAKRISHNAN ANUSHA SUBRAMANIAN ET AL: "Understanding the biocalorimetric and respirometric behaviour of co-culture (R. eutropha, P. putida and A. vinelandii) in poly (3-hydroxybutyrate) batch production", BIOCHEMICAL ENGINEERING JOURNAL, ELSEVIER, AMSTERDAM, NL, vol. 155, 7 August 2019 (2019-08-07), XP086008808, ISSN: 1369-703X, DOI: 10.1016/J.BEJ.2019.107334 [retrieved on 2019-08-07]
- Nakahara Hiroki ET AL: "Induction of spontaneous phenotype conversion in Ralstonia solanacearum by addition of iron compounds in liquid medium - ScienceDirect", , 6 May 2021 (2021-05-06), pages 1-20, XP055809346, Retrieved from the Internet: URL:https://www.sciencedirect.com/science/ article/pii/S0167701221001019 [retrieved on 2021-05-31]
- MATTHIAS RABERG ET AL: "Ralstonia eutropha H16 in progress: applications beside PHAs and establishment as production platform by advanced genetic tools", CRITICAL REVIEWS IN BIOTECHNOLOGY,, vol. 38, 12 December 2017 (2017-12-12), pages 494-510, XP002792845,

## Description

### Technical field

The present invention relates to a co-culture system comprising two different strains of the bacterium *Ralstonia eutropha (Cupriavidus necator)* and its use for production of value-added compounds.

### Background

Co-culture techniques find broad applications and are of great importance in synthetic biology, as multi-species cell consortia are widely seen to hold enormous potential for production of both novel and known industrial or medical compounds and as these systems have shown to have increased productivity and other advantages over their respective monocultures (Bernstein et al., 2012).

In some cases, strains cannot easily be monocultured as the strain does not exhibit desired *in vivo* physiological behaviours, such as the efficient ability to metabolize the giving substrate and/or to synthesize the product of interest, but the presence of another cell population may improve the culturing success or cell behaviour, such as by conversion of complex substrate or removal of growth-inhibiting effects of accumulating metabolites or medium compounds.

For example, polyhydroxyalkanoates (PHAs), a group of natural biodegradable polyesters with broad industrial applications, have been produced with a single PHA-producing bacterial strain from widely available carbon sources such as sucrose, or hydrolysed sucrose-rich substrates (Jian Yu, 2016; Sen et al., 2019). However the process usually ends by a complete consumption of one sugar (glucose) while the second accumulates (fructose). A difference in the consumption rate of these two sugars by a PHA accumulating strain has been reported before (Raberg et al 2011; Dalsasso et al 2019). This selective consumption of an individual sugar not only affects the substrate yield, but the accumulated higher sugar concentration also inhibits the growth and PHA productivity of the bacterial strain.

A well-known microbial production platform strain is the soil bacterium *Ralstonia eutropha,* also known as *Cupriavidus necator.* The wild-type strain *Ralstonia eutropha* H16 (*Cupriavidus necator* H16, DSM 428) is known to produce and sequester PHA plastics when exposed to excess amounts of sugar substrate, *Ralstonia eutropha* H16 has been used as platform for industrial production of PHAs and single cell protein. Because of the wide spectrum of substrate utilization and ability to grow to high cell density, this strain has been intensively studied and prepared during last decades to be a robust microbial cell factory, similarly to other microbial strains already used at industrial level such as *Saccharomyces cerevisiae, Corynebacterium glutamicum* or *Escherichia coli. Ralstonia eutropha* H16 is capable of both autotrophic and heterotrophic growth, it utilizes gases like CO₂, H₂, O₂ and syngas, short chain organic acids, sugars, fatty acids and lipids and aromatic compounds. In addition to the continued isolation of new strains of the species *R. eutropha* from different environments, the availability of genome sequences, mutagenesis, expression vectors and promotor systems have enabled proper engineering of these strains to produce many biotechnological products such as biopolymers like polythioesters, medium-chain length poly(3-hydroxyalkanoates), poly(2-hydroxyalkanoates), poly(4-hydroxyalkanoates), cyanophycin, and value-added chemicals such as (R)-hydroxyalkanoic acids, 2-methylcitric acid, and different alcohols and alkenes such as butanol, isobutanol, or isopropanol. The strain is also used for bioenergy production like in fuel-cells, light-driven hydrogen, or the production of non-alcohol biofuels molecules like the methyl esters of polyhydroxyalkanoate, or methyl ketones. Moreover, *R. eutropha* has great potential to be a potent host strain for recombinant protein expression system (Lu et al. 2016; Raberg et al. 2018).

An even wider spectrum of substrates can be utilized by the mutated or engineered strains of *R. eutropha* especially the mixed sugars or short and medium chain fatty acids those produced from the hydrolysis of agro- and food-industries such as cellulose- fat-, and sucrose-rich streams. Co-culturing *R. eutropha* strain with other microbial or bacterial species such as bacillus, pseudomonas or saccharomyces has been studied to co-utilize a mixture of substrates and to maximize the yield and productivity of the process (Rebocho et al. 2020; Tan et al. 2014). Further, Bhatia S. K., Bioresource Technology, 257 (2018), 92-101 discloses two Ralstonia eutropha strains, namely Ralstonia eutropha H16 and Ralstonia eutropha 5119, which were each co-cultured with a sucrose-hydrolysing microbe, namely Bacillus subtilis and Bacillus amyloliquefaciens, to optimise the production of polyhydroxyalkonate (PHA) and the generation of biomass. However, as microbial strains from different species differ widely in their required and optimum growth media and conditions, it is extremely challenging to provide a strain that is not only compatible with the growth and medium conditions of the given mono-cultured strain, but is also able to increase its production of the desired compound(s) to an extent greater than with mono-culture. Additionally, any microbial strains possibly found compatible for co-culturing are likely to produce a range of other non-desired metabolites, which can have undesired effects, such as requiring extra steps to separate these compounds apart.

There thus exists an unmet need for improved co-culture systems for the culturing of strains or variants of the species *Cupriavidus necator* that addresses these issues of culture conditions, optimized substrates consumption, separation of co-produced metabolites and enhanced productivity of the compound(s) of interest.

### Summary

The invention relates to a co-culture system of two bacterial strains belonging to the species *Cupriavidus necator (Ralstonia eutropha),* the two strains differing for their capabilities of utilizing and/or metabolizing different carbon sources. The invention is defined by the claims. Any subject matter falling outside the scope of the claims is provided for information purposes only.

The present inventors have in fact found that a fructose-utilizing C. *necator,* such as C. *necator* H16 (DSM 428) and a glucose-utilizing C. *necator,* such as C. *necator* H1G⁺3 (DSM 545), can be cultivated together using hydrolysed sucrose as carbon source. C. *necator* DSM 428 is the wild-type and uses fructose as carbon source, while C. *necator* DSM 545, which is a mutant of C. *necator* H16, prefers using glucose over fructose as carbon source. Such a co-culture system can ensure higher product yield and higher productivity compared to culturing either strain alone. The higher productivity is obtained by avoiding the growth-inhibiting effect of the accumulated and not being utilized sugar by the bacteria. PHA yield of 70 g/L of cell dry weight and even higher have been obtained with the disclosed co-culture system.

The co-culture system and the process disclosed herein allow optimal conversion of the total raw material to final product. Thus, waste material, for example residual fructose or glucose, is minimized.

One embodiment of the present invention is directed to a co-culture system for production of single-cell protein material, pyruvate and/or pyruvate derived products, the co-culture system comprising a microbial component and a substrate,
wherein the microbial component comprises or consists of a first and a second microbial cell, wherein the first and the second microbial cells are different strains of the species *Cupriavidus necator,* wherein the substrate comprises at least a first carbon source and a second carbon source, the first carbon source being different from the second carbon source,
wherein the first microbial cell is capable of metabolizing the first carbon source,
wherein the second microbial cell is capable of metabolizing the second carbon source, and wherein said pyruvate derived products are:
   growth-associated metabolites selected from alcohols, biofuels and/or amino acids; and/or
   homo- and co-polymeric polyhydroxyalkanoates, co-polymeric polythioesters, monomeric hydroxyalkanoates and/or cyanophycin.

One aspect of present disclosure relates to a process for production of single-cell protein material of *Cupriavidus necator,* said process comprising:
a) providing a feedstock;
b) providing a microbial component of co-culture system as defined herein; and
c) contacting the feedstock with the microbial component of the co-culture system, thereby fermenting the feedstock to obtain a fermented composition comprising pyruvate and/or pyruvate derived products,
wherein the pyruvate derived products are growth-associated metabolites selected from alcohols, biofuels and/or amino acids; and/or homo- and co-polymeric polyhydroxyalkanoates, co-polymeric polythioesters, monomeric hydroxyalkanoates and/or cyanophycin.

One embodiment of the present invention relates to a process for production of single-cell protein material, pyruvate and pyruvate derived products, said process comprising:
a) providing a feedstock;
b) providing a microbial component of co-culture system as defined herein; and
c) contacting the feedstock with the microbial component of the co-culture system, thereby fermenting the feedstock to obtain a fermented composition comprising pyruvate and/or pyruvate derived products,
wherein the pyruvate derived products are growth-associated metabolites selected from alcohols, biofuels and/or amino acids; and/or homo- and co-polymeric polyhydroxyalkanoates, co-polymeric polythioesters, monomeric hydroxyalkanoates and/or cyanophycin.

One aspect of the present disclosure is directed to a kit of parts for production of single-cell protein material, pyruvate and/or pyruvate derived products, the kit of parts comprising or consisting of a first and a second microbial cell and instructions for use, wherein the first and the second microbial cells are different strains of the species *Cupriavidus necator,*
wherein the first microbial cell is capable of metabolizing a first carbon source, and
wherein the second microbial cell is capable of metabolizing a second carbon source,
wherein the first carbon source differs from the second carbon source, and wherein the pyruvate derived products are growth-associated metabolites selected from alcohols, biofuels and/or amino acids; and/or homo- and co-polymeric polyhydroxyalkanoates, co-polymeric polythioesters, monomeric hydroxyalkanoates and/or cyanophycin.

One aspect of the present disclosure is directed to a system for production of single-cell protein material, pyruvate and/or pyruvate derived products, said system comprising a fermenting reactor configured for performing the process for microbial production of growth-associated metabolites like pyruvate and/or pyruvate derived products described herein, hence by using the co-culture system described herein, wherein the pyruvate derived products are growth-associated metabolites selected from alcohols, biofuels and/or amino acids; and/or homo- and co-polymeric polyhydroxyalkanoates, co-polymeric polythioesters, monomeric hydroxyalkanoates and/or cyanophycin.

One aspect of the present disclosure relates to a composition comprising native polyhydroxyalkanoate granules obtained by the process disclosed herein. The PHA bioplastics accumulates as intracellular granules, which can be bioextracted and recovered in their native granular form.

One aspect of the present disclosure relates to a use of native polyhydroxyalkanoate granules obtained by the process disclosed herein for the manufacture of a bioplastic.

One aspect of the present disclosure relates to a use of the composition comprising native polyhydroxyalkanoate granules disclosed herein for the manufacture of a bioplastic.

### Description of Drawings

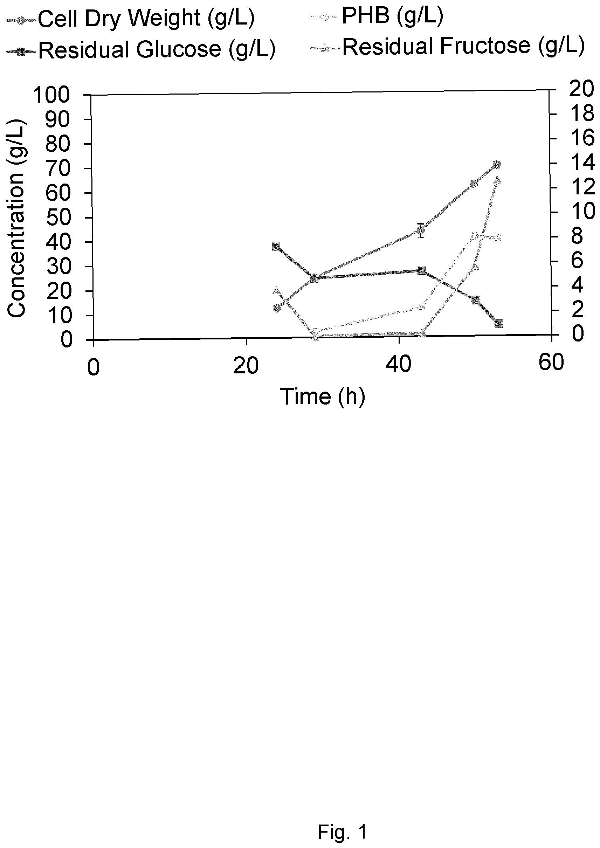

**Figure 1** Fed-batch fermentation with mono-culture *C*. *necator* DSM 545 for PHB production. A mono-culture of *C*. *necator* DSM 545 was fed with hydrolysed sucrose (a mixture of fructose and glucose). Cells grew to a relatively high cell density and accumulated PHB, however fructose started to accumulate during the continued feeding.

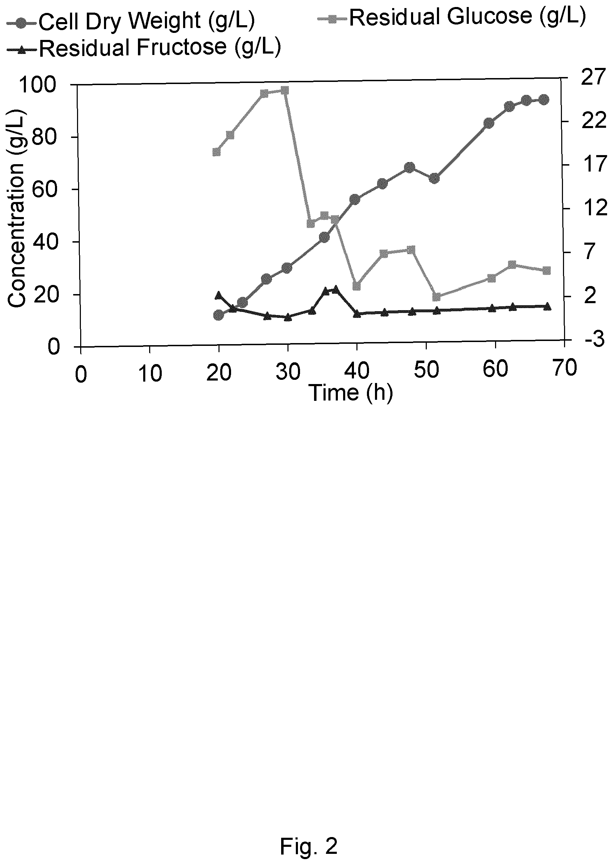

**Figure 2** Fed-batch fermentation with co-culture *C*. *necator* DSM 428 and *C*. *necator* DSM 545 for PHB production from hydrolysed sucrose. A co-culture of the tested glucose- and fructose-utilizing strains succeeded to consume both glucose and fructose and enabled the fed-batch fermentation to reach higher cell density and higher PHB content compared to the mono-culture.

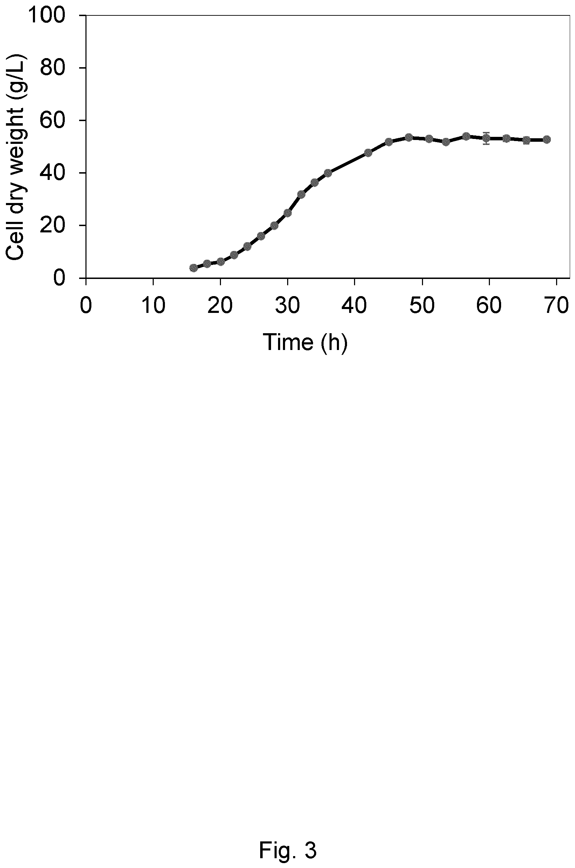

**Figure 3** Fed-batch fermentation with mono-culture *C*. *necator* DSM 545 for PHBV production from hydrolysed sucrose. A mono-culture of *C*. *necator* DSM 545 was also used for production of the copolymer PHBV with continuous feeding of hydrolysed sucrose and the co-substrate propionate. The glucose utilizing strain grew to relatively high cell density and accumulated PHBV to some extent, however, higher cell density could not be achieved despite feeding continued.

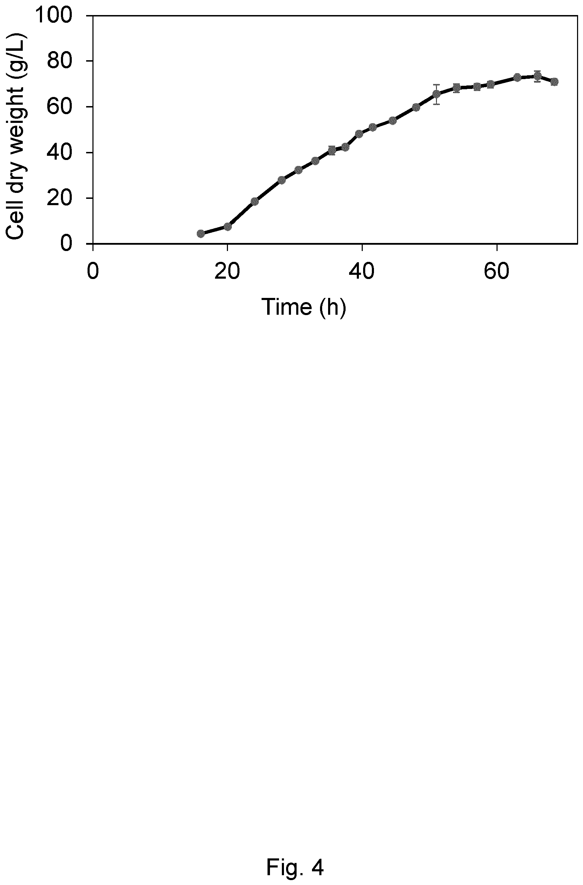

**Figure 4** Fed-batch fermentation with co-culture *C*. *necator* DSM 428 and *C*. *necator* DSM 545 for PHBV production from hydrolysed sucrose. The co-culture system reached higher cell density and higher PHBV productivity while feeding of hydrolysed sucrose and the co-substrate continued, compared to the *C*. *necator* DSM 545 mono-culture.

### Detailed description

### Definitions

The term "microbial cell" as used herein means cells from a microorganism, in particular from a bacterium. Preferably, "a microbial cell" is used to refer to a plurality of microbial cells of the same microbial (bacterial) strain.

The term "carbon source" as used herein is equivalent to "fermentable carbon source" and means a carbon source that a microorganism will metabolize to derive energy. Typical carbon sources include, but are not limited to, monosaccharides, oligosaccharides, polysaccharides, alkanes, fatty acids, esters of fatty acids, monoglycerides, diglycerides, triglycerides, carbon dioxide, ethanol, methanol, formaldehyde, formate and carbon-containing amines. Each of C. *necator* strains may have the ability to use a single carbon source for the production of a product and for its growth, thus that single carbon source is referred to as "sole carbon source". A C. *necator* strain that uses for example glucose or fructose or a fatty acid as the sole carbon source, is also able to grow on other carbon sources. The meaning of the phrase " sole carbon source" means that other carbon sources also may be present.

The term "capable of metabolizing" is used in the present disclosure relative to carbon sources. The term "capable of metabolizing" as used herein means that a microbial cell is capable of both uptaking a certain carbon source and deriving energy from it though its metabolic systems. For example, wild type *C*. *necator (C. necator* DSM 428, DSM 428) cannot uptake glucose but has nevertheless the enzymes required to derive energy from it; hence wild type *C*. *necator (C. necator* DSM 428, DSM 428) is not capable of metabolizing glucose. Other *C*. *necator* strains may be able to uptake glucose but lack the enzymes required to derive energy from it; hence these strains are not capable of metabolize glucose. The mutant *C*. *necator* H1G⁺3 (DSM 545) is capable of both uptaking and deriving energy from glucose, hence it is capable of metabolizing glucose.

The term "titer" or "titer of a compound" refers herein to the produced concentration of a compound. When the compound is produced by a cell, the term refers to the total concentration produced by the cell, i.e. the total amount of the compound divided by the volume of the culture medium.

As used herein, "high cell density" refers to a cultivation which yields a high number of microbial prokaryotic or eukaryotic cells in a defined volume of culture medium and a defined period of cultivation. The high-cell-density value is dependent on the microbial cell and can be defined as the cell density value that is reached with gradual addition of growth-limiting substrates (fed-batch) without intoxicating the microbe

The term "biofuel" refers to a fuel in which all carbon contained within the fuel is derived from biomass, such as a plant or an animal waste, and is biochemically converted, at least in part, into a fuel by a microorganism. Biofuels include, but are not limited to, biodiesel, biohydrogen, biogas, biomass-derived dimethylfuran (DMF), and the like. In particular, the term "biofuel" can be used to refer to plant-derived alcohols, such as ethanol, methanol, propanol, or butanol, which can be denatured, if desired prior to use. The term "biofuel" can also be used to refer to fuel mixtures comprising plant-derived fuels, such as alcohol/gasoline mixtures (i.e., gasohols). The biofuel can be any biofuel produced by aerobic or anaerobic fermentation of plant material, such as sugar beets and sugar cane molasses.

The term "single-cell protein material" or "SCP material" refers to proteins derived from micro-organisms, such as yeast and bacterial proteins, for inclusion in animal diets. For example, "single-cell protein material may be microbial biomass, such as edible bacterial biomass. Bacterial biomass is in fact rich in protein. Single-cell microorganisms such as fungi, yeasts and bacteria have been proposed as new sources of protein in human foods and as certain animal feeds. These may be grown by single-cell reproduction. Today, the most widely used protein-containing microorganisms (also referred to as "single-cell protein material") are those derived from fungi or yeast. The present disclosure relates in some aspects to the production of single-cell proteins derived from bacteria, in particular from a co-culture of two different strains of the species *Cupriavidus necator.*

### Co-culture system and kit

One aspect of the present disclosure is directed to a co-culture system for production of single-cell protein material, pyruvate and/or pyruvate derived products,
the co-culture system comprising a microbial component and a substrate,
wherein the microbial component comprises or consists of a first and a second microbial cell,
wherein the first and the second microbial cells are different strains of the species
*Cupriavidus necator,*
wherein the substrate comprises at least a first carbon source and a second carbon source, the first carbon source being different from the second carbon source,
wherein the first microbial cell is capable of metabolizing the first carbon source, and
wherein the second microbial cell is capable of metabolizing the second carbon source, and wherein said pyruvate derived products are growth-associated metabolites selected from alcohols, biofuels and/or amino acids; and/or homo- and co-polymeric polyhydroxyalkanoates, co-polymeric polythioesters, monomeric hydroxyalkanoates and/or cyanophycin.

In one embodiment, the present disclosure is directed to a co-culture system for production of polyhydroxyalkanoates,
the co-culture system comprising a microbial component and a substrate,
wherein the microbial component comprises or consists of a first and a second microbial cell,
wherein the first and the second microbial cells are different strains of the species
*Cupriavidus necator,*
wherein the substrate comprises at least a first carbon source and a second carbon source, the first carbon source being different from the second carbon source,
wherein the first microbial cell is capable of metabolizing the first carbon source, and
wherein the second microbial cell is capable of metabolizing the second carbon source.

In one embodiment, the present disclosure is directed to a co-culture system for production of single-cell protein material of *Cupriavidus necator,*
the co-culture system comprising a microbial component and a substrate,
wherein the microbial component comprises or consists of a first and a second microbial cell,
wherein the first and the second microbial cells are different strains of the species
*Cupriavidus necator,*
wherein the substrate comprises at least a first carbon source and a second carbon source, the first carbon source being different from the second carbon source,
wherein the first microbial cell is capable of metabolizing the first carbon source, and
wherein the second microbial cell is capable of metabolizing the second carbon source.

In fact, the present invention has found that two strains of the bacterial species *Cupriavidus necator* having similar, but not identical, metabolism, can be cultivated together at the same culture conditions and result in improved production of certain valuable compounds while at the same time they can better utilize the substrate available in the culture, especially if that substrate comprises more than one carbon source. In fact, wild-type *Cupriavidus necator* is not capable of metabolizing glucose because it is not capable of uptaking glucose from the growth medium (even it may has the enzymes required for deriving energy from glucose) (Raberg et al 2011), but a number of glucose-utilizing *Cupriavidus necator* mutant strains have been isolated and/or obtained through UV irradiation (Schlegel and Gottschalk 1965), chemical mutagenesis (Kim et al. 1995), and metabolic engineering (Sichwart et al. 2011).

The present invention has found that co-culture of a *Cupriavidus necator* strain that is capable of metabolizing glucose together with a *Cupriavidus necator* strain that is not capable of metabolizing glucose is possible and even advantageous, both for improving substrate utilization and for producing certain microbial products at a higher titer compared to when a *Cupriavidus necator* strain is cultured alone with the same substrate.

The present disclosure is directed to a co-culture of two strains belonging to the *Cupriavidus necator* species. The names *Cupriavidus necator* and *Ralstonia eutropha* are used interchangeably herein.

*Cupriavidus necator* is a Gram-negative soil bacterium of the class Betaproteobacteria. *Cupriavidus necator* has gone through a series of name changes. C. *necator* was originally named *Hydrogenomonas eutrophus* because it fell under the Hydrogenomonas classification. After characterizing cell morphology, metabolism and GC content, the *Hydrogenomonas* nomenclature was disbanded because it comprised many species of microorganisms. *H. eutrophus* was then renamed *Alcaligenes eutropha* because it was a micro-organism with degenerated peritrichous flagellation. Investigating phenotype, lipid composition, fatty acid composition and 16S rRNA analysis, *A. eutropha* was found to belong to the genus *Ralstonia* and named *Ralstonia eutropha.* Upon further study of the genus, Ralstonia was found to comprise two phenotypically distinct clusters. The new genus *Wautersia* was created from one of these clusters which included *R. eutropha.* In turn *R. eutropha* was renamed *Wautersia eutropha.* Looking at DNA-DNA hybridization and phenotype comparison with *Cupriavidus necator, W. eutropha* was found to be the same species as previously described C. *necator.* Because C. *necator* was named in 1987 far before the name change to *R. eutropha* and *W. eutropha,* the name C. *necator* was assigned to *R*. *eutropha* according to Rule 23a of the International Code of Nomenclature of Bacteria. Currently, *Cupriavidus necator* is still referred to as *Ralstonia eutropha.*

In one embodiment of the present disclosure, the microbial component of the co-culture system comprises or consists of a first and a second microbial cell, wherein the second microbial cell is a mutant or a variant of the first microbial cell. The mutant microbial cell may be a genetically engineered mutant and/or a mutant isolated from a pool of mutants obtained by induced mutagenesis of the wild-type and/or a mutant isolated from nature, where spontaneous mutagenesis occurs. In particular, the first and second microbial cells differ in their carbon-metabolism; for example, they may have preferred a certain carbon source over other carbon sources, or they may not be able to metabolize a certain carbon source.

In one embodiment of the present disclosure, the first and the second microbial cells are each a plurality of microbial cells.

In one embodiment of the present disclosure, the microbial component consists of the first and the second microbial cell.

In one embodiment of the present disclosure, the ratio of first microbial cell to second microbial cell is 1:1, such as 1:2, such as 1:3, such as 1:4, such as 4:1, such as 3:1, such as 3:2. For example the ratio of first microbial cell to second microbial cell can be adjusted depending on the composition of the substrate or the strain growth rate, such as depending on the carbon sources present in the substrate, in a way to optimize substrate utilization and consumption.

In one embodiment of the present disclosure, the first microbial cell is not capable of metabolizing the second carbon source. This is because the first microbial cell may be, for example, not capable of uptaking that carbon source from the environment, and/or because it lacks the enzymes required to derive energy from that carbon source. For example, the first microbial cell may be a wild-type cell of *Cupriavidus necator,* for example *Cupriavidus necator* DSM 428, which is not capable of metabolizing glucose.

If such a microbial cell is used in a mono-culture with a substrate that contains and/or fed with fructose and glucose, the glucose in the culture will not be used and can instead accumulate in the growth (fermentation) medium, such as in fed-batch fermentation, and reach concentrations (usually >20 g/L) which inhibit cell growth and product formation. As a consequence of the inhibition effect, other carbon sources and nutrients being fed to the medium will also accumulate because the cells stop growing and consuming the nutrients.

In one embodiment of the present disclosure, the first and the second microbial cells are polyhydroxyalkanoate (PHA)-producing microbial cells. *Cupriavidus necator* is known for being able to produce PHAs under nitrogen-starvation and by using monosaccharides, gases and/or fatty acids as precursors.

In one embodiment of the present disclosure, the first and the second microbial cells are mesophiles. Hence, they are cultured at conditions which can easily be maintained.

In one embodiment of the present disclosure, the substrate comprises at least two carbon sources, wherein the first carbon source is fructose and/or wherein the second carbon source is glucose. Thus, at least one of the two *Cupriavidus* necator strains can metabolize fructose, and at least one of the two *Cupriavidus necator* strains can metabolize glucose. For example, one *Cupriavidus necator* strain may be capable of metabolizing glucose but not fructose, or one *Cupriavidus necator* strain may be capable of metabolizing fructose but not glucose, or one *Cupriavidus necator* strain may be capable of metabolizing glucose and fructose.

Thus, in one embodiment of the present disclosure, the first microbial cell is not capable of metabolising glucose.

Thus, in one embodiment of the present disclosure, the second microbial cell is capable of metabolising glucose.

In one embodiment of the present disclosure, the first microbial cell is capable of metabolising fructose.

In one embodiment of the present disclosure, the first microbial cell is capable of converting fructose into pyruvate and/or pyruvate derived products, such as of producing single-cell material by metabolizing fructose. In one embodiment of the present disclosure, the first microbial cell is capable of converting fructose into primary metabolites, or growth-associated metabolites, such as pyruvate, alcohols, biofuels and/or amino acids; and/or into secondary metabolites such as homo- and co-polymeric polyhydroxyalkanoates, co-polymeric polythioesters, monomeric hydroxyalkanoates, cyanophycin and/or other valuable products.

In one embodiment of the present disclosure, the first microbial cell is not capable of using glucose for its growth, and thus of using glucose for production of single-cell protein material, and of converting glucose into pyruvate and/or pyruvate derived products. This is because the first microbial cell is, for example, not capable of uptaking glucose from the environment, and/or because it lacks the enzymes required to derive energy from glucose.

In one embodiment of the present disclosure, the second microbial cell is capable of using glucose for its growth, and thus of using glucose for production of single-cell protein material, and of converting glucose into pyruvate and/or pyruvate derived products. In one embodiment of the present disclosure, the second microbial cell is capable of converting glucose into biomass, pyruvate and pyruvate derived products, wherein said pyruvate derived products are primary metabolites, or growth-associated metabolites, such as, alcohols, biofuels and/or amino acids; and/or secondary metabolites, such as homo- and co-polymeric polyhydroxyalkanoates, co-polymeric polythioesters, monomeric hydroxyalkanoates and/or cyanophycin.

In one embodiment of the present disclosure, the second microbial cell has a reduced capability compared to the first microbial cell to convert fructose into biomass, such as of using fructose for its own growth and thus for production of single-cell protein material, as well as to convert fructose into growth associated products such as pyruvate and/or pyruvate derived products.

In one embodiment of the present disclosure, the first and the second microbial cells are capable of converting pyruvate into a monopolymeric polyhydroxyalkanoate (PHA), a co-polymeric polyhydroxyalkanoate (PHA), a co-polymeric polythioester (PTE) and/or an (R)-hydroxyalkanoate monomer.

In one embodiment of the present disclosure, the first and the second microbial cells are capable of converting pyruvate into a monopolymeric polyhydroxyalkanoate (PHA), medium-chain length poly(3-hydroxyalkanoates), poly(2-hydroxyalkanoates), poly(4-hydroxyalkanoates), poly(3-hydroxybutyrate) (P(3HB)), a co-polymeric polyhydroxyalkanoate (PHA), P(3HB-co-3HV), P(3HB-co-3HV-co-4HV), P(3HB-co-3HHx), a co-polymeric polyhydroxyalkanoate (PHA) and polythioester (PTE) and/or an (R)-hydroxyalkanoate monomer, or a combination thereof.

In one embodiment of the present disclosure, the first and the second microbial cells are capable of converting pyruvate into cyanophycin.

In one embodiment of the present disclosure, the first and the second microbial cells are capable of converting pyruvate into 2-methylcitric acid, butanol, isobutanol, or isopropanol, methyl esters of polyhydroxyalkanoate, and/or methyl ketones, and/or other value-added products.

The capability of a microbial cell to metabolize a carbon source, for example glucose and/or fructose, can be determined by culturing said microbial cell in a medium comprising the specific carbon source as sole carbon source, for example glucose or fructose, and measuring its concentration over time by HPLC, thereby determining that the carbon source is being consumed, thus metabolized, if its concentration decreases overtime. In addition, the increase in concentration overtime of biomass and product, as well as of growth by-products or gases, such as CO₂, can also be monitored, for example by monitoring optical density (OD) overtime, where an increase in OD indicates that the carbon source is being used by the microbial cell to grow and produce biomass. HPLC protocols for determining the residual concentration of a carbon source, such as of a monosaccharide, such as of glucose or fructose in a sample are known in the art. An example is given in Example 1 of the present application.

The capability of a microbial cell to convert a carbon source, for example glucose and/or fructose, into polyhydroxyalkanoates can be determined by culturing said microbial cell in a medium comprising the specific carbon source as sole carbon source preferably under nitrogen-limiting conditions, for example glucose or fructose, and measuring the concentration of polyhydroxyalkanoates in the cell biomass over time by gas chromatography (GC), thereby determining that polyhydroxyalkanoates are being produced as a product from the sugars being metabolised. GC protocols for determining the concentration of specific polyhydroxyalkanoates, for example of polyhydroxybutyrate, are known in the art. An example is given in Example 2 of the present application.

In one embodiment of the present disclosure, the first microbial cell is *Cupriavidus necator* DSM 428. Hence, in one embodiment of the present disclosure, the first microbial cell is a wild-type *Cupriavidus necator.*

In one embodiment of the present disclosure, the second microbial cell is *Cupriavidus necator* DSM 545. Hence, in one embodiment of the present disclosure, the second microbial cell is a mutant of the wild-type *Cupriavidus necator,* such as of *Cupriavidus necator* DSM 428, wherein said mutant is capable of metabolizing glucose.

In one embodiment of the present disclosure, said co-culture system is for production of homo- and co-polymeric polyhydroxyalkanoates, co-polymeric polythioesters, monomeric hydroxyalkanoates, alcohol and non-alcohol biofuels, and/or others valuable products.

One aspect of the present disclosure is directed to a system for production of single-cell protein material, pyruvate and/or pyruvate derived products, said system comprising a fermenting reactor configured for performing the process for microbial production of single-cell protein material, such as microbial biomass, or growth associated products, such as pyruvate and/or pyruvate derived products described herein, hence by using the co-culture system described herein, wherein the pyruvate derived products are growth-associated metabolites selected from alcohols, biofuels and/or amino acids; and/or homo- and co-polymeric polyhydroxyalkanoates, co-polymeric polythioesters, monomeric hydroxyalkanoates and/or cyanophycin.

One aspect of the present disclosure is directed to a kit of parts for production of single-cell protein material, pyruvate and/or pyruvate derived products, the kit of parts comprising or consisting of a first and a second microbial cell and instructions for use, wherein the first and the second microbial cells are different strains of the species *Cupriavidus necator,*
wherein the first microbial cell is capable of metabolizing a first carbon source, and
wherein the second microbial cell is capable of metabolizing a second carbon source,
wherein the first carbon source differs from the second carbon source,
wherein the pyruvate derived products are growth-associated metabolites selected from alcohols, biofuels and/or amino acids; and/or homo- and co-polymeric polyhydroxyalkanoates, co-polymeric polythioesters, monomeric hydroxyalkanoates
and/or cyanophycin.

In one embodiment of the present disclosure, the first and the second carbon sources that can be metabolized by the first and the second microbial cells present in the kit of parts are different organic carbon sources selected from the group consisting of glucose, fructose, propionic acid, valeric acid, levulinic acid, formic acid, butyric acid, citric acid, acetic acid, CO₂, syngas, short and/or medium chain fatty acid, mercaptoalkanoate, glycerol, gluconic acid, xylose, N-acetyl-d-glucosamine (NAG), benzoate, phenol, ethanol, methanol, 3-mercaptopropionic acid (3MP), 3-mercaptovaleric acid (3MV), 3,3'-thiodipropionic acid (TDP), 3,3'-dithiodipropionic acid (DTDP) and 3-mercaptovaleric acid (3MV).

In one embodiment of the present disclosure, the first and the second carbon sources that can be metabolized by the first and the second microbial cells present in the kit of parts are fructose and glucose

In one embodiment of the present disclosure, the first and the second microbial cells are each a plurality of microbial cells.

In one embodiment of the present disclosure, the first and the second microbial cells are polyhydroxyalkanoate (PHA)-producing microbial cells.

In one embodiment of the present disclosure, the second microbial cell is a mutant of the first microbial cell.

In one embodiment of the present disclosure, first microbial cell is *Cupriavidus necator* DSM 428 and the second microbial cell is a *Cupriavidus necator* strain capable of metabolizing glucose.

In one embodiment of the present disclosure, the first microbial cell is *Cupriavidus* necator strain capable of using fructose as sole carbon source, and the second microbial cell is a *Cupriavidus necator* strain capable of using a fatty acid as sole carbon source.

In one embodiment of the present disclosure, the first microbial cell is *Cupriavidus necator* DSM 428, and the second microbial cell is a *Cupriavidus necator* strain capable of using a fatty acid, such as a short-chain fatty acid or a medium-chain fatty acid, as sole carbon source.

In one embodiment of the present disclosure, the first microbial cell is a *Cupriavidus* necator strain capable of using a fatty acid, such as a short-chain fatty acid or a medium-chain fatty acid, as sole carbon source and/or wherein the second microbial cell is a *Cupriavidus* necator strain capable of using glucose as sole carbon source.

In one embodiment of the present disclosure, the first microbial cell is a *Cupriavidus necator* strain capable of using a fatty acid as sole carbon source and/or wherein the second microbial cell is *Cupriavidus necator* DSM 545.

In one embodiment of the present disclosure, the first microbial cell is *Cupriavidus necator* DSM 428 and the second microbial cell is *Cupriavidus necator* DSM 545.

*C. necator* DSM 428, the wild-type strain that utilizes fructose but not glucose, and C. *necator* DSM 545, a mutant of the strain DSM 428 which is able to utilize glucose and fructose. These two strains are remarkably similar being the wild-type and mutant of the same strain, and their co-culture results in better consumption of the carbon sources, in particular fructose and glucose, and higher titer of polyhydroxyalkanoates, compared to mono-culture of each of the strains alone.

Recombinant C. *necator* strains exist, that prefer short-chain or medium-chain fatty acid as carbon source. These strains are useful in producing co-polymeric PHAs when part of a co-culture as described herein.

In one embodiment of the present disclosure, the kit of parts further comprises medium components which are suitable for cultivation of said microbial cells.

In one embodiment of the present disclosure, the instructions for use of the kit of parts comprise cultivation recipe.

### Pyruvate derived products and single-cell protein material

*Cupriavidus necator* is very effective in converting pyruvate to polyhydroxyalkanoates, nevertheless *Cupriavidus necator* can also convert pyruvate to other valuable products, for example primary metabolites or growth-associated metabolites, or even simply biomass, which can be used as single-cell protein material.

Pyruvate derived products are all those products that can be obtained by *Cupriavidus necator* when further metabolizing pyruvate for example via AcetylCoA and acetoacetyl

CoA production, or via the valine biosynthesis pathway. This is well explained in Lu et al. 2016 (in particular Fig. 12.7). Thus, examples of pyruvate derived products are homo- and co-polymeric polyhydroxyalkanoates, co-polymeric polythioesters, monomeric hydroxyalkanoates, alcohol and non-alcohol biofuels, amino acids, cyanophycin and/or others valuable products.

More in particular, polyhydroxyalkanoates that can be produced by the systems and processes disclosed herein are poly-3-hydroxybutyrate (PHB, P(3HB)), polyhydroxyvalerate (PHV, P(3HV)), polyhydroxyhexanoate (PHHx, P(3HHx)) and/or poly(3-hydroxybutyrate-co-3-hydroxyvalerate)(PHBV, P(3HB-co-3HV), poly(3-hydroxybutyrate-co-3-hydroxyvalerate-co-4-hydroxyvalerate)(PHBVV, P(3HB-co-3HV-co-4HV)), poly(3-hydroxybutyrate-co-3-hydroxyhexanoate)(PHBHx, P(3HB-co-3HHx), co-polymers of polythioesters (co-PTE), poly(3-hydroxybutyrate-co-3-mercaptopropionate (P(3HB-co-3MP)), or a combination thereof.

Similarly, monomeric hydroxyalkanoates that can be produced by the systems and processes disclosed herein are (R)-hydroxybutyrate, (R)-hydroxyvalerate, and/or (R)-hydroxyhexanoate.

Similarly, biofuels that can be produced by the systems and processes disclosed herein are: alcohol biofuels such as butanol, isobutanol, 3-methyl-1-butanol, and/or isopropanol; but also non-alcohol biofuels such as diesel additives, such as ketones. Polythioesters co-polymers can be produced by the systems and processes disclosed herein, in particular when the substrate comprises in addition to hydrolyzed sucrose, also one or more of 3-mercaptopropionic acid (3MP), 3-mercaptovaleric acid (3MV), 3,3'-thiodipropionic acid (TDP), 3,3'-dithiodipropionic acid (DTDP) and 3-mercaptovaleric acid (3MV).

In one embodiment of the present disclosure, polythioesters co-polymers produced by the systems and processes disclosed herein are poly(3-hydroxybutyrate-co-3-mercaptopropionate) [P(HB-co-3MP)] and other PTE copolymers having thiol-containing precursors substrates.

One aspect of the present disclosure relates to a composition comprising native polyhydroxyalkanoate granules obtained by the process disclosed herein. The native granules produced according to the process of the present disclosure can be used to prepare stable suspensions for water-based coating of paper and different fibrous materials. The native granules produced according to the process of the present disclosure can also be used for fine biotechnological applications such as matrix for protein purifications (functionalized bioactive beads). (Grage et al. 2009)

The native granules produced according to the process of the present disclosure can also be used as replacements for fossil-based microbeads in personal care applications, as texture enhance or as functionalized micro and nanobeads carrying different formulations.

One aspect of the present disclosure relates to a use of native polyhydroxyalkanoate granules obtained by the process disclosed herein for the manufacture of a bioplastic item or formulation.

In one embodiment of the present disclosure, polyhydroxyalkanoates produced according to the process disclosed herein, or using a co-culture system disclosed herein can be used in any of the following applications: as exfoliants and film forming in personal care with ability to load formulations; in packaging materials, including for food; as mulch films in agriculture; as slow-release fertilizer and pesticide carriers; in single-use plastic items such as hygiene products, razors, utensils, diapers, shopping bags, cotton swab sticks, cubs, cutlery, plates, and straws; as fibers in textiles, for example non-woven fabrics; in biomedical applications (implants for tissue engineering, wound dressing, and surgical items); in pharmaceutical applications for both packaging and drug delivery, including slow release/long-term dosage) (Williams and Martin 2005); in moulded goods, paper coatings, and as performance additives; for uses such as injection moulding, extrusion, thermoforming, foaming, 3D-printing, coating of paper and other materials, glues, adhesives, as additive for reinforcement/barrier enhancer or plasticisation or as building block for thermosets in paints and foams; in electronics, furniture, cars, and buildings. Applications of polyhydroxyalkanoates are known to the person of skill in the art as found for example in 'The Handbook of Polyhydroxyalkanoates - Postsynthetic Treatment, Processing and Application' (2020)".

One aspect of the present disclosure relates to a use of the composition comprising native polyhydroxyalkanoate granules disclosed herein for the manufacture of a bioplastic or for the manufacture of PHA-monomer.

One aspect of the present disclosure relates to a use of single-cell protein material of *Cupriavidus necator* obtained by the process disclosed herein for use in a feed or food for animals, and/or into an ingredient for cosmetics and/or personal care products. The process disclosed herein and/or a co-culture system disclosed herein results in production of co-products that can be extracted and/or recovered. For example, emulsifying, foaming and gelling ingredients for use in feed and/or food; whole products or fractions, for example separated oligopeptides, lipids or carbohydrates with certain molecular size or free amino acids, fatty acids, nucleic acids, nucleotides, or sugars for various uses; proteins and bioactive compounds obtained from non-PHA biomass hydrolysate for use in food, feed and/or personal care.

### Process for production of pyruvate and pyruvate derived products

One embodiment of the present invention relates to a process for production of single-cell protein material, pyruvate and/or pyruvate derived products, said process comprising:
a) providing a feedstock;
b) providing a microbial component of co-culture system as defined herein; and
c) contacting the feedstock with the microbial component of the co-culture system, thereby fermenting the feedstock to obtain a fermented composition comprising pyruvate and/or pyruvate derived products,
wherein the pyruvate derived products are growth-associated metabolites selected from alcohols, biofuels and/or amino acids; and/or homo- and co-polymeric polyhydroxyalkanoates, co-polymeric polythioesters, monomeric hydroxyalkanoates and/or cyanophycin.

In particular, the co-culture system is a co-culture system comprising a microbial component and a substrate,
wherein the microbial component comprises or consists of a first and a second microbial cell,
wherein the first and the second microbial cells are different strains of the species *Cupriavidus necator,*
wherein the substrate comprises at least a first carbon source and a second carbon source, the first carbon source being different from the second carbon source,
wherein the first microbial cell is capable of metabolizing the first carbon source, and
wherein the second microbial cell is capable of metabolizing the second carbon source.

The pyruvate derived products are growth-associated metabolites selected from alcohols, biofuels and/or amino acids; and/or homo- and co-polymeric polyhydroxyalkanoates co-polymeric polythioesters, monomeric hydroxyalkanoates and/or cyanophycin.

In one embodiment of present disclosure, the feedstock is put in contact with the microbial component of the co-culture system in presence of a cultivation medium, wherein said cultivation medium may comprise a nitrogen source, such as ammonium hydroxide, and/or other salts and minerals that are required for cultivation of *Cupriavidus necator.*

One embodiment of present disclosure relates to a process for production of polyhydroxyalkanoates, said process comprising:
a) providing a feedstock;
b) providing a microbial component of co-culture system as defined herein; and
c) contacting the feedstock with the microbial component of the co-culture system, thereby fermenting the feedstock to obtain a fermented composition comprising polyhydroxyalkanoates.

One embodiment of present disclosure relates to a process for production of single-cell protein material of *Cupriavidus necator,* said process comprising:
a) providing a feedstock;
b) providing a microbial component of co-culture system as defined herein; and
c) contacting the feedstock with the microbial component of the co-culture system, thereby fermenting the feedstock to obtain a fermented composition comprising single-cell protein material of *Cupriavidus necator.*

One embodiment of present disclosure relates to a process for production of polyhydroxyalkanoates and single-cell protein material of *Cupriavidus necator,* said process comprising:
a) providing a feedstock;
b) providing a microbial component of co-culture system as defined herein; and
c) contacting the feedstock with the microbial component of the co-culture system, thereby fermenting the feedstock to obtain a fermented composition comprising polyhydroxyalkanoates and single-cell protein material of *Cupriavidus necator.*

In one embodiment of the present disclosure, the inoculum size, such as the concentration of the two microbial cells of the co-culture system, is between 1% v/v and 5% v/v of the total fermentation composition, such as between 1%v/v and 4% v/v, such as between 1% v/v and 3% v/v, such as between 1% v/v and 2% v/v, such as between 2 % v/v and 3% v/v, such as between 2% v/v and 4% v/v, such as between 2% v/v and 5% v/v, such as between 3% v/v and 4% v/v, such as between 3% v/v and 5% v/v, such as between 4% v/v and 5% v/v of the total fermentation volume/composition. The total fermentation composition comprises the inoculum, the feedstock, additional salt and nutrients, and water.

The process disclosed herein comprises a fermentation step (step c) during which the co-culture disclosed herein converts two or more carbon sources present in the feedstock into single-cell protein material, such as biomass, of *Cupriavidus necator* and/or value-added products, such as pyruvate and/or pyruvate derived products, which can be homo- and co-polymeric polyhydroxyalkanoates, co-polymeric polythioesters, monomeric hydroxyalkanoates, alcohol and non-alcohol biofuels, and/or other valuable products.

The fermentation step can for example be conducted via a fed-batch process.

In one embodiment of the present disclosure, the two microbial cells which are in the co-culture system are put in contact with the feedstock at different times. For example, in one embodiment of the present disclosure the first microbial cell of the co-culture system is put in contact with the feedstock first, and the second microbial cell of the co-culture system is put in contact with the feedstock later. In one embodiment of the present disclosure the first microbial cell and the second microbial cell of the co-culture system are put in contact with the feedstock at the same time.

Consequently, in one embodiment of the present disclosure, the first microbial cell and the second microbial cell of the co-culture system may be in contact with the feedstock together and thus co-fermenting the substrate, for 12 h or more, such as for 15 h or more, such as for 18 h or more, such as for 20 h or more such as for 24 h or more, such as for 28 h or more, such as for 36 h or more, such as for 40 h or more, such as for 44 h or more, such as for 48 h or more, such as for 52 h or more, such as for 60 h or more. In some embodiments, the first microbial cell is added prior to the second microbial cell; in some other embodiments, the second microbial cell is added prior to the first microbial cell. The first and the second microbial cells are nevertheless cultured together for a certain amount of time as defined herein.

The time of addition of the first and the second microbial cell, as well as the co-culturing time are optimized based on the carbon source content of the feedstock in a way to obtain high consumption rate of the carbon source and with the aim to utilize all carbon source, for example all glucose and all fructose, in the feedstock.

In one embodiment of the present disclosure, step c) comprises a first phase that allows growth of the microbial component of the co-culture system, this growth phase is also characterized by production of pyruvate and other primary metabolites, such as alcohols, biofuels and/or amino acids, as well as accumulation of microbial cells, and a second phase that allows production of pyruvate derived products, for example homo- and co-polymeric polyhydroxyalkanoates, co-polymeric polythioesters, monomeric hydroxyalkanoates and/or cyanophycin.

In one embodiment of the present disclosure, the phase that allows growth of the microbial component of the co-culture system is characterized by presence of substrate comprising two or more carbon sources and a cultivation medium comprising other nutrients required for growth of the microbial component of co-culture system, including one or more nitrogen source, salts and minerals that may not be present in the substrate. The nitrogen source can for example be ammonium hydroxide.

In one embodiment of the present disclosure, the phase that allows production of Z homo- and co-polymeric polyhydroxyalkanoates, co-polymeric polythioesters, monomeric hydroxyalkanoates and/or cyanophycin is characterized by feeding of a cultivation medium being poor in certain nutrients. For example, when polyhydroxyalkanoates are to be produced, the phase in the fermentation in step c) that allows production of the polyhydroxyalkanoates is characterized by the cultivation medium being poor in nitrogen. In fact, nitrogen starvation is usually needed for the co-culture disclosed herein to efficiently produce polyhydroxyalkanoates.

In one embodiment of the present disclosure, ammonium hydroxide is fed to the fermentation during the phase that allows growth of the microbial component and production of pyruvate and growth-associated metabolites selected from alcohols, biofuels and/or amino acids but not during the phase that allows production of PHAs.

In one embodiment of the present disclosure, ammonium hydroxide is fed to the fermentation both during the phase that allows growth of the microbial component and production of pyruvate and growth-associated metabolites selected from alcohols, biofuels and/or amino acids and during the phase that allows production of co-polymeric polythioesters, monomeric hydroxyalkanoates and/or cyanophycin

In one embodiment of the present disclosure, the phase that allows growth of the microbial component is characterized by a continuous feeding rate with a feedstock comprising hydrolysed sucrose or hydrolysed sucrose and valerate and a cultivation medium comprising a nitrogen source such as ammonium hydroxide.

In one embodiment of the present disclosure, the phase of step c) that allows growth of the microbial component and production of growth-associated metabolites selected from alcohols, biofuels and/or amino acids is characterized by a continuous feeding rate of the feedstock and of the cultivation medium for 24 h to 38 h, such as 28 h to 38 h, such as 30 h to 38 h, such as 32 h to 38 h, such as for 34 h to 38 h, such as 36 h to 38 h, such as 24 h to 34 h, such as 24 h to 32 h, such as 24 h to 30 h, such as 24 h to 28 h, such as 24 h to 26 h.

In one embodiment of the present disclosure, the phase of step c) that allows production of the polyhydroxyalkanoates is characterized by application of nitrogen limited conditions after about 24 h of inoculation, such as after about 32 h of inoculation, such as after about 34 h of inoculation, such as after about 36 h of inoculation, such as after about 38 h of inoculation, such as after about 40 h of inoculation, such as after about 42 h of inoculation, such as after about 44 h of inoculation, such as after about 46 h of inoculation, such as after about 48 h of inoculation, such as after about 30 to 38 h of inoculation. "Inoculation" as used herein is the time at which the microbial component, for example both the first and the second microbial cells at the same time, or at least one of the first or the second microbial cells, is put in contact with the feedstock.

In one embodiment of the present disclosure, the phase of step c) that allows production of the polyhydroxyalkanoates is characterized by continuous feeding of the feedstock and of the cultivation medium, wherein the cultivation medium does not comprise a nitrogen source, such as does not comprise ammonium hydroxide.

In one embodiment of the present disclosure, the phase of step c) that allows production of the polyhydroxyalkanoates is characterized by continuous feeding of the feedstock and of the cultivation medium, wherein the cultivation medium does not comprise a nitrogen source, for 24 h to 38 h, such as 28 h to 38 h, such as 30 h to 38 h, such as 32 h to 38 h, such as for 34 h to 38 h, such as 36 h to 38 h, such as 24 h to 34 h, such as 24 h to 32 h, such as 24 h to 30 h, such as 24 h to 28 h, such as 24 h to 26 h.

One embodiment of present disclosure relates to a process for production of homo- and co-polymeric polyhydroxyalkanoates, co-polymeric polythioesters, monomeric hydroxyalkanoates, said process comprising:
a) providing a feedstock;
b) providing a microbial component of a co-culture system as defined herein; and
c) i) contacting the feedstock with the two microbial cells of the microbial component simultaneously or sequentially as described herein,
c) ii) applying a continuous feeding rate of feedstock and cultivation medium, wherein the cultivation medium comprises a nitrogen source, for 24 to 38 h,
c) iii) applying a continuous feeding rate of feedstock and a nitrogen-poor cultivation medium for 12 to 48 h, thus applying nitrogen limiting conditions,
thereby fermenting the feedstock to obtain a fermented composition comprising homo- and co-polymeric polyhydroxyalkanoates, co-polymeric polythioesters and/or monomeric hydroxyalkanoates..

One embodiment of present disclosure relates to a process for production of pyruvate and/or pyruvate derived products, wherein the pyruvate derived products are growth-associated metabolites selected from alcohols, biofuels and/or amino acids; and/or homo- and co-polymeric polyhydroxyalkanoates, co-polymeric polythioesters, monomeric hydroxyalkanoates and/or cyanophycin, said process comprising:
a) providing a feedstock;
b) providing a microbial component of a co-culture system as defined herein; and
c)
   i) contacting the feedstock with the two microbial cells of the microbial component simultaneously or sequentially as described herein,
   ii) applying a continuous feeding rate of feedstock and cultivation medium, wherein the cultivation medium comprises a nitrogen source, for 24 to 38 h, such as for 24 to 48 h, such as for 32 to 56 h, such as for 30 to 62 h, such as for no longer than 96 h;
thereby fermenting the feedstock to obtain a fermented composition comprising pyruvate and/or pyruvate derived products, wherein the pyruvate derived products are growth-associated metabolites selected from alcohols, biofuels and/or amino acids; and/or homo- and co-polymeric polyhydroxyalkanoates, co-polymeric polythioesters, monomeric hydroxyalkanoates and/or cyanophycin.

One of the advantages of the process disclosed herein is that it allows to reach high cell density (HCD) and high product content, for example high polyhydroxyalkanoates content. Hence, in one embodiment of the present disclosure, a product of the process, or a co-product (for example when PHAs are also produced and recovered) of the process is biomass itself, such as single-cell protein, of *Cupriavidus necator,* which is also a valuable product and can be used as described herein.

In one embodiment of the present disclosure, the feedstock provided in the process disclosed herein comprises one or more carbon sources selected from the group consisting of glucose, fructose, propionic acid, valeric acid, levulinic acid, formic acid, butyric acid, citric acid, acetic acid, CO₂, syngas, short and/or medium chain fatty acid, mercaptoalkanoate, glycerol, gluconic acid, xylose, N-acetyl-d-glucosamine (NAG), benzoate, phenol, ethanol, methanol, 3-mercaptopropionic acid (3MP), 3-mercaptovaleric acid (3MV), 3,3'-thiodipropionic acid (TDP), 3,3'-dithiodipropionic acid (DTDP) and 3-mercaptovaleric acid (3MV).

In one embodiment of the present disclosure, the raw material comprises or consists of molasses, such as sucrose-rich materials, and wherein the raw material derives from sugarcane and/or sugar beets. Molasses, such as sugarcane and/or sugar beets molasses, are commonly used to obtain sucrose - sugar - for the food industry. However, producers all over the world are looking for alternative and more valuable products that can be obtained from molasses. Fermentation of molasses, and in particular sucrose, by microorganisms like *Cupriavidus necator* opens up to a large variety of products obtainable from sucrose, such as pyruvate and pyruvate derived products as defined herein.

In one embodiment of the present disclosure, the feedstock provided in the process disclosed herein comprises glucose and fructose.

In one embodiment of the present disclosure, the feedstock is hydrolyzed sucrose. For example, the feedstock may be hydrolyzed molasses.

In one embodiment of the present disclosure, the process further comprises a pre-treatment step comprising hydrolysing a raw material comprising sucrose to obtain the feedstock.

In one embodiment of the present disclosure, the raw material comprises a concentration of sucrose of 40% w/v or above, such as 50% w/v or above, or such as 60% w/v or above, such as 70% w/v or above, such as about 70% w/v.

In one embodiment of the present disclosure, the feedstock comprises a concentration of glucose of 20% w/v or above, such as 25% w/v or above or such as 30% w/v or above, such as 35% w/v or above, such as about 35% w/v.

In one embodiment of the present disclosure, the feedstock comprises a concentration of fructose of 20% w/v or above, such as 25% w/v or above or such as 30% w/v or above, such as 35% w/v or above, such as about 35% w/v.

In one embodiment of the present disclosure, glucose and fructose represent at least the 60%, such the at least the 70% such as at least the 80%, such as at least the 90%, such as at least the 95%, such as about 100% of the organic carbon in the feedstock. Hence, glucose and fructose may be the sole carbon sources available to the *Cupriavidus necator* strains of the co-culture system of the present disclosure.

However, in one embodiment of the present disclosure, the feedstock comprises other carbon sources in addition to glucose and fructose. For example, the feedstock may also comprise any of propionic acid, valeric acid, levulinic acid, formic acid, butyric acid, citric acid, acetic acid, CO₂, syngas, short and/or medium chain fatty acid, mercaptoalkanoate, glycerol, gluconic acid, xylose, N-acetyl-d-glucosamine (NAG), benzoate, phenol, ethanol, methanol, 3-mercaptopropionic acid (3MP), 3-mercaptovaleric acid (3MV), 3,3'-thiodipropionic acid (TDP), 3,3'-dithiodipropionic acid (DTDP) and 3-mercaptovaleric acid (3MV). These additional carbon sources may be present in the feedstock depending on how the raw material is hydrolysed, for example depending on which temperature and pH are used during thermo-acid hydrolysis, or depending on the time length of the hydrolysis of the raw material. These additional carbon sources may also be added to the feedstock for the purpose of the process, as the co-culture system of the present disclosure may advantageously utilize any of the additional carbon sources together with glucose and fructose to produce certain pyruvate derived products. For example, if levulinic acid (4-ketovaleric acid) is present in the feedstock, the co-culture system disclosed herein may use it for production of poly(3-hydroxybutyrate-co-3-hydroxyvalerate)(PHBV) and/or poly(3-hydroxybutyrate-co-3-hydroxyvalerate-co-4-hydroxyvalerate)(P(3HB-co-3HV-co-4HV).

One of the advantages of the process of the present disclosure is that it allows consumption of the sugars present in the feedstock at a similar rate, in equal manner, and completely, or almost completely since the fed-batch mode used for the fermentation implies that new feedstock is added at regular time intervals. In particular, when the feedstock comprises for the most part glucose and fructose, the process of the present disclosure allows equal consumption of the two substrates, thus avoiding problems of substrate accumulation. Hence, the process of the present disclosure allows for an efficient use of all the carbon source present in the feedstock, thanks to the co-culture system described herein.

In one embodiment of the present disclosure, the glucose concentration in the fermented composition, thus at the end of the process, is of 20 g/l or less, such as of 15 g/L or less, such as of 10 g/L or less, such as of 5 g/L or less, such as of 1 g/L or less.

In one embodiment of the present disclosure, the fructose concentration in the fermented composition is of 20 g/l or less, such as of 15 g/L or less, such as of 10 g/L or less, such as of 5 g/L or less, such as of 1 g/L or less.

In one embodiment of the present disclosure, the concentration of fructose and glucose together (the sum of their concentration) in the fermented composition is of 20 g/l or less, such as of 15 g/L or less, such as of 10 g/L or less, such as of 5 g/L or less, such as of 1 g/L or less.

In one embodiment of the present disclosure, the glucose and the fructose are the sole sources of carbon for the microbial cells.

A feedstock suitable for the process of the present disclosure can be obtained by hydrolyzing certain raw materials, such as molasses and other sucrose-rich raw materials. Hence, in one embodiment of the present disclosure, the process comprises a pre-treatment step in which the raw material is treated, usually hydrolyzed, to become the feedstock. Methods for hydrolyzing raw materials such as molasses and other sucrose-rich raw materials, are known in the art.

In one embodiment of the present disclosure, the hydrolysis of the raw material in the pre-treatment step is thermo-acidic hydrolysis, or biological hydrolysis. The characteristics of the feedstock may vary depending on the conditions used for hydrolysis of the raw material. For example, if thermo-chemical hydrolysis is used, higher temperature and longer hydrolysis time can further convert part of the fructose into levulinic acid, formic acid, acetic acid and HMF. Levulinic acid can lead to co-polymer production, for example production of PHBVV. In case of biological hydrolysis of sucrose, a specific enzyme catalyses the reaction and therefore the hydrolysed sucrose comprises or consists of the monosugars (glucose and fructose) in equivalent molar ratio.

In one embodiment of the present disclosure, the hydrolysis of the raw material in the pre-treatment step is a biological hydrolysis, and utilizes enzymes, such as the enzyme beta-fructofuranosidase, active or rested bacterial or yeast cells, such as rested *Saccharomyces cerevisiae* cells comprising the enzyme beta-fructofuranosidase in their cell wall.

In one embodiment of the present disclosure, the raw material comprises sucrose and one or more carbon sources selected from the group consisting of glucose, fructose, propionic acid, valeric acid, levulinic acid, formic acid, butyric acid, citric acid, acetic acid, CO₂, syngas, short and/or medium chain fatty acid, mercaptoalkanoate, glycerol, gluconic acid, xylose, N-acetyl-d-glucosamine (NAG), benzoate, phenol, 3-mercaptopropionic acid (3MP), ethanol, methanol, 3-mercaptovaleric acid (3MV), 3,3'-thiodipropionic acid (TDP), 3,3'-dithiodipropionic acid (DTDP) and 3-mercaptovaleric acid (3MV).

In one embodiment of the present disclosure, one or more carbon sources selected from the group consisting of glucose, fructose, propionic acid, valeric acid, levulinic acid, formic acid, butyric acid, citric acid, acetic acid, CO₂, syngas, short and/or medium chain fatty acid, mercaptoalkanoate, glycerol, gluconic acid, xylose, N-acetyl-d-glucosamine (NAG), benzoate, phenol, ethanol, methanol, 3-mercaptopropionic acid (3MP), 3-mercaptovaleric acid (3MV), 3,3'-thiodipropionic acid (TDP), 3,3'-dithiodipropionic acid (DTDP) and 3-mercaptovaleric acid (3MV) are added to the raw material after or during the pre-treatment step.

In one embodiment of the present disclosure, the temperature of the fermenting step c) is between 25°Cand 35°C, such as between 27°C and 33°C, such as between 29°C and 31°C, such as about 30°C.

In one embodiment of the present disclosure, pH of the fermenting step c) is between pH 6 and pH 9, such as between pH 6.5 and 8.5, such as between pH 6.7 and 8.2.

In one embodiment of the present disclosure, the total time for fermentation in step c) is 36 h to 96 h, such as 36 h to 84 h, such as 48 h to 84 h, such as 36 h to 68 h, such as 48 h to 68 h, such as 48h to 72h, such as about 62 h. The total fermentation time may be divided in a first phase and a second phase, wherein the first phase comprises feeding of feedstock and cultivation medium, the cultivation medium comprising a nitrogen source, and wherein the second phase comprises feeding of feedstock and a nitrogen-poor cultivation medium. In some embodiments of the present disclosure, feeding of feedstock and cultivation medium, the cultivation medium comprising a nitrogen source, occurs for the whole fermentation time.

In one embodiment of the present disclosure, the dissolved oxygen for fermentation at step c) is between 0% v/v and 60% v/v of saturation, such as between 5% v/v and 60% v/v of saturation, such as between 10% v/v and 60% v/v, such as between 10% v/v and 40% v/v, such as between 10% v/v and 30% v/v, such as about 20% v/v of saturation.

To control the dissolved oxygen concentration during fermentation, compressed air may be supplied. Thus, in one embodiment of the present disclosure the fermenting step c) comprises compressed air and/or oxygen supply between 0.1 and 2.0 vvm, such as between 0.5 and 2.0 vvm (volume/volume/minute), such as between 0.5 and 1.0 vvm.

In one embodiment of the present disclosure, the fermenting step c) comprises mechanical stirring. This helps both in keeping a certain dissolved oxygen concentration, and in keeping a homogeneous distribution of cells, substrates, oxygen and other nutrients within the fermentation composition.

In one embodiment of the present disclosure, step a) further comprises a step of sterilizing the feedstock. For example, sterilization may be performed by autoclaving, pasteurization, pascalization, ionizing radiation or UV radiation.

In one embodiment of the present disclosure, the pyruvate derived product obtained by the process disclosed herein is a monomeric hydroxyalkanoate, a polyhydroxyalkanoate (PHA) and/or a polythioester (PTE) co-polymer.

In one embodiment of the present disclosure, the process further comprises a step d) of recovering the monomeric hydroxyalkanoate, the polyhydroxyalkanoate (PHA) the polythioester (PTE) co-polymer and/or a the single-cell protein material.

In one embodiment of the present disclosure, recovering the polyhydroxyalkanoates and/or the polythioesters co-polymers in step d) comprises lysing the microbial cells in the fermented composition by bacterial cells from the species *Bacillus pumilus;* centrifugation and/or filtration. In particular, it is advantageous to recover the polyhydroxyalkanoates and/or the polythioesters co-polymers with help of bacterial cells from the species *Bacillus pumilus,* as described in detail in WO 2016/085396, since this method is solvent-free, cost effective and allows recovery of polyhydroxyalkanoates and/or the polythioesters in native granules form. This extraction method converts the non-PHA biomass into hydrolysed biomass, which can be used as single cell protein (SCP) hydrolysate. Thus, two or more products can be recovered from the same process, and used for various applications, as described herein in the present disclosure.

In one embodiment of the present disclosure, the fermented composition comprises polyhydroxyalkanoates, wherein the polyhydroxyalkanoates produced comprise or consist of poly-3-hydroxybutyrate (PHB), polyhydroxyvalerate (PHV), polyhydroxyhexanoate (PHH) and/or poly(3-hydroxybutyrate-co-3-hydroxyvalerate)(PHBV) and/or poly(3-hydroxybutyrate-co-3-hydroxyvalerate-co-4-hydroxyvalerate)(P(3HB-co-3HV-co-4HV)), or a combination thereof..

In one embodiment of the present disclosure, the fermented composition comprises polyhydroxyalkanoates, wherein the polyhydroxyalkanoates produced comprise poly-3-hydroxybutyrate (PHB).

In one embodiment of the present disclosure, the fermented composition comprises polyhydroxyalkanoates, wherein the polyhydroxyalkanoates produced comprise polyhydroxyvalerate (PHV).

In one embodiment of the present disclosure, the fermented composition comprises polyhydroxyalkanoates, wherein the polyhydroxyalkanoates produced comprise polyhydroxyvalerate (PHV) co-polymer.

In one embodiment of the present disclosure, the fermented composition comprises polyhydroxyalkanoates, wherein the polyhydroxyalkanoates produced comprise polyhydroxyhexanoate (PHHx).

In one embodiment of the present disclosure, the fermented composition comprises polyhydroxyalkanoates, wherein the polyhydroxyalkanoates produced comprise polyhydroxyhexanoate (PHHx) co-polymer.

In one embodiment of the present disclosure, the fermented composition comprises polyhydroxyalkanoates, wherein the polyhydroxyalkanoates produced comprise poly(3-hydroxybutyrate-co-3-hydroxyvalerate)(PHBV).

In one embodiment of the present disclosure, the fermented composition comprises polyhydroxyalkanoates, wherein the polyhydroxyalkanoates produced comprise poly(3-hydroxybutyrate-co-3-hydroxyvalerate-co-4-hydroxyvalerate)(P(3HB-co-3HV-co-4HV)).

In one embodiment of the present disclosure, the fermented composition comprises polyhydroxyalkanoates, wherein the polyhydroxyalkanoates produced consist of poly-3-hydroxybutyrate (PHB).

In one embodiment of the present disclosure, the fermented composition comprises polyhydroxyalkanoates, wherein the polyhydroxyalkanoates produced consist of polyhydroxyvalerate (PHV).

In one embodiment of the present disclosure, the fermented composition comprises polyhydroxyalkanoates, wherein the polyhydroxyalkanoates produced consist of polyhydroxyhexanoate (PHH).

In one embodiment of the present disclosure, the fermented composition comprises polyhydroxyalkanoates, wherein the polyhydroxyalkanoates produced consist of poly(3-hydroxybutyrate-co-3-hydroxyvalerate)(PHBV).

In one embodiment of the present disclosure, the fermented composition comprises polyhydroxyalkanoates, wherein the polyhydroxyalkanoates produced consist of poly(3-hydroxybutyrate-co-3-hydroxyvalerate-co-4-hydroxyvalerate)(P(3HB-co-3HV-co-4HV)).

In one embodiment of the present disclosure, the polyhydroxyalkanoates produced have an average molecular weight of at least 100 kDa, such as between 100 kDa and 2000 kDa, such as between 200 kDa and 2000 kDa, such as between 300 kDa and 2000 kDa, such as between 400 kDa and 1500 kDa, such as between 500 kDa and 1000 kDa, such as between 800 kDa and 2000 kDa, such as between 500 kDa and 1500 kDa.

In one embodiment of the present disclosure, the polyhydroxyalkanoates produced have an average chain length of 3 to 6 carbons, such as an average chain length of 4 to 5 carbons, such as an average chain length of 3 carbons, such as an average chain length of 4 carbons, such as an average chain length of 5 carbons, such as an average chain length of 6 carbons.

In one embodiment of the present disclosure, the polyhydroxyalkanoates produced are a co-polymer of poly(3-hydroxybutyrate-co-3-hydroxyvalerate)(PHBV) having a content of 3-hydroxyvalerate of between 5 mol% and 40 mol%, such as of between 5 mol% and 35 mol%, such as of between 5 mol% and 30 mol%, such as of between 5 mol% and 25 mol%, such as of between 5 mol% and 20 mol%, such as of between 5 mol% and 15 mol%, such as of between 5 mol% and 10 mol%, such as of between 10 mol% and 20 mol%, such as of between 15 mol% and 20 mol%, such as of between 10 mol% and 30 mol%, such as of between 20 mol% and 40 mol%, such as of between 15 mol% and 35 mol%, such as of between 10 mol% and 25 mol%.

In one embodiment of the present disclosure, the polyhydroxyalkanoates produced are a homopolymer of poly(3-hydroxybutyrate) and a co-polymer of poly(3-hydroxybutyrate-co-3-hydroxyvalerate)(PHBV) having a content of 3-hydroxyvalerate of between 5 mol% and 40 mol%, such as of between 5 mol% and 35 mol%, such as of between 5 mol% and 30 mol%, such as of between 5 mol% and 25 mol%, such as of between 5 mol% and 20 mol%, such as of between 5 mol% and 15 mol%, such as of between 5 mol% and 10 mol%, such as of between 10 mol% and 20 mol%, such as of between 15 mol% and 20 mol%, such as of between 10 mol% and 30 mol%, such as of between 20 mol% and 40 mol%, such as of between 15 mol% and 35 mol%, such as of between 10 mol% and 25 mol%.

In one embodiment of the present disclosure, the polyhydroxyalkanoates are native polyhydroxyalkanoate granules with intact molecular weight and crystallinity.

In one embodiment of the present disclosure, the process disclosed herein comprising using the co-culture system disclosed herein allows production of polyhydroxyalkanoates at a higher titer compared to a mono-culture of a *Cupriavidus* necator strain, provided the same culture conditions are used.

In one embodiment of the present disclosure, the titer of polyhydroxyalkanoates produced is at least 3 g/L, such as at least 8 g/L, such as at least 10 g/L, 15 g/L, such as at least 20 g/L, such as at least 25 g/L, such as at least 30 g/L, such as at least 35 g/L, such as at least 40 g/L, such as at least 45 g/L, such as at least 50 g/L, such as at least 55 g/L, such as at least 60 g/L, such as at least 65 g/L, such as at least 70 g/L, such as at least 73 g/L, such as at least 75 g/L, such as at least 80 g/L, such as at least 85 g/L, such as at least 90 g/L, such as at least 91 g/L, such as at least 95 g/L, such as at least 100 g/L, such as at least 110 g/L, such as at least 125 g/L, such as at least 135 g/L, such as at least 150 g/L.

In one embodiment of the present disclosure, the polyhydroxyalkanoates produced constitute at least 50% w/w, such as at least 55% w/w, such as at least 60% w/w, such as at least 65% w/w, such as at least 70% w/w, such as at least 75% w/w, such as at least 80% w/w, such as at least 85% w/w, such as at least 90% w/w of the cell dry weight.

In one embodiment of the present disclosure, the process disclosed herein further comprising a step of processing the polyhydroxyalkanoate. For example, poly-3-hydroxybutyrate (PHB), polyhydroxyvalerate (PHV), polyhydroxyhexanoate (PHH) and/or poly(3-hydroxybutyrate-co-3-hydroxyvalerate)(PHBV) and/or poly(3-hydroxybutyrate-co-3-hydroxyvalerate-co-4-hydroxyvalerate)(P(3HB-co-3HV-co-4HV)), or a combination thereof. For example, the polyhydroxyalkanoate can be used to make polymer resins, latex, and/or film materials for use in packaging, coating, textiles, furniture, and more.

In one embodiment of the present disclosure, the pyruvate derived product is a biofuel selected from the group consisting of butanol, isobutanol, 3-methyl-1-butanol, and isopropanol.

In one embodiment of the present disclosure, the process disclosed herein further comprises a step d) of recovering the butanol, isobutanol, 3-methyl-1-butanol, and/or isopropanol.

In one embodiment of the present disclosure, the product of the process disclosed herein is single-cell protein material of *Cupriavidus necator.*

Thus, in one embodiment of the present disclosure, the process disclosed herein comprises recovering the single-cell protein material from the fermented composition in step d) by using bacterial cells from the species *Bacillus pumilus.* This recovery method is disclosed in Ibrahim et al., 2020, WO 2016/085396.

In one embodiment of the present disclosure, the recovered single cell protein material is used in feed or food for animals, and/or as an ingredient for cosmetics and/or personal care products.

### Examples

### Example 1 - Hydrolysis of sucrose-rich substrates into its mono-sugars

### Materials and methods

Three different sucrose-rich substrates from sugar beet industry were used, molasses, thick juice, and crystalline sucrose (sucrose content of 45 - 49, 70 - 75, and 99 - 100% w/v, respectively). The substrates were hydrolysed at the following conditions: thermo-acidic hydrolysis at pH 2.6 - 3.0, and controlled temperature 121°C, for a period of40, 60, or 80 minutes. Substrate preparations of Molasses and thick juice were hydrolysed with their original concentration (100% w/v). Crystalline sucrose was hydrolysed at 70% w/v concentration in water.

Other sucrose hydrolysis methods can be used such as enzyme hydrolysis (invertase, EC 3.2.1.26) or invertase in active or rested bacterial or yeast cells can be used as an alternative biobased method.

Analysis of sugars was done using high-performance liquid chromatography (HPLC) system (Agilent HPLC 1260 Infinity II Quaternary System) consisting of a Bio-Rad Aminex HPX-87H column operated at 50°C and using 5 mM sulphuric acid as mobile phase with a flow rate of 0.5 mL/min. The system was equipped with a refractive index detector, RID. Quantitative analysis was done against standard solutions of defined concentration of analytical grade sucrose (ACROS Organics, Belgium), fructose (Alfa Aesar), and glucose (VWR Chemicals).

### Results:

At the optimized hydrolysis conditions (121°C for 40 min with initial pH adjusted to 2.6 - 2.8), a complete conversion of sucrose in the prepared solution (70% w/v) was achieved, 97 - 100% w/v (HPLC analysis), corresponding to the equivalent concentrations of its monosugars, glucose and fructose (1:1 w/w). The same hydrolysis method was applied to all sucrose-rich substrates (molasses and thick-juice).

### Example 2 - Cultivation and inoculum preparation of strain DSM 428 and DSM 545 for mono-and co-culture fermentations of Cupriavidus necator strains

### Materials and methods:

The strains used in the current study were *Cupriavidus necator* DSM 545 and *Cupriavidus necator* DSM 428 (formerly known as *Alcaligenes eutrophus, Ralstonia eutropha* and *Wautersia eutropha*).

The cultivation conditions and media used for the two strains were optimized to achieve simultaneous growth and polymer synthesis.

The following media were used in the different stages of the process:
- Activation and pre-culture medium (Tryptic soy broth, TSB, OXOID Ltd., England): This medium was only used to activate the microorganisms prior to preparing the seed cultures; TSB was supplemented with 1 g/L glucose during the activation of both strains *C*. *necator* DSM 428 and *C*. *necator* DSM 545. Agar (16 g/L) was added for solid culture preparation in plates.

- Seed medium (mineral salts medium, MSM-1): This medium was used to cultivate both strains individually in flasks to achieve appropriate inoculum of each strain which will be able to grow optimally in the production medium either in flasks or in bioreactor. MSM-1 contains the following components in gram/liter: Na₂HPO₄.12H₂O, 9; KH₂PO₄, 1.5; NH₄Cl, 1.0 (as the main nitrogen source); MgSO₄.7H₂O, 0.2; NH₄ Fe(III)-citrate, 0.0012; CaCl₂.2H₂O, 0.02. MSM-1 was supplemented with 1 mL of trace elements solution-1 (TES-1) containing the following components in grams/liter: EDTA, 50.0; FeCl₃, 8.3; ZnCI2, 0.84; CuCl₂.2H₂O, 0.13; CoCl₂.6H₂O, 0.1; MnCl₂.6H₂O, 0.016; and H₃BO₃, 0.1. The monosugars were added separately after sterilization, fructose for *C*. *necator* DSM 428, and glucose for *C*. *necator* DSM 545. Culture pH was adjusted aseptically after sterilization. (Schlegel etal., 1961; Ibrahim and Steinbüchel, 2009)
- Production medium (MSM-2) (Mozumder et al. 2014): This medium was used for growth and polymer synthesis by the two strains either in mono- or co-culture systems. MSM-2 contains the following components in gram/liter: Citric acid, 1.87; KH₂PO₄, 13.3; (NH₄)₂SO₄, 4.0 (as nitrogen source); MgSO₄.7H₂O, 1.2. The components were supplemented with 10 mL of trace elements solution-2 (TES-2) containing the following components in gram/liter: H₃BO₃, 0.093; FeSO₄.7H₂O, 10; ZnSO₄.7H₂O, 2.25; MnSO₄.7H₂O, 0.5; (NH₄)₆Mo₇O₂₄, 0.1; CaCl₂.2H₂O, 0.2; CuSO₄.5H₂O, 1.0. Trace elements solution also contained 10 ml/L concentrated HCI 37%. Sugar substrates or hydrolysed sucrose-rich substrates (prepared as described in Example 1), were added separately after sterilization. Culture pH was adjusted aseptically after sterilization.
- Preparation of the co-culture inoculum (seed): Prior to inoculation in seed medium, both strains were activated individually on solid TSB in plates for 20-24h. Subsequently, a single colony from activated plate of each strain was used separately to inoculate two TSB pre-culture flasks containing 1 g/L glucose, one flask for each strain. After TSB pre-culture has grown for 24h, relatively similar optical density of each strain was achieved by both strains (OD₆₀₀ 12 - 16). The seed cultures of the two strains (MSM-1) were inoculated individually using 5%(v/v) inoculum from TSB pre-cultures into Erlenmeyer flasks 250-mL or 1-L with 50 or 200 ml of the seed medium, MSM-1, respectively, supplemented with individual substrate. After 24-26 h incubation at 30°C and stirring at 180 rpm, growth of both wild-type *C*. *necator* DSM 428 and C. *necator* DSM 545 reached similar optical density (OD₆₀₀ 12 - 16).

The optimized inoculums were used individually or together to test the growth of the wild type strain and the mutant strain individually or in a co-culture mode. Cultivation was done in shake flasks with MSM-2, supplemented with one sugar or both pure sugars, or the hydrolysis product of sucrose-rich substrates. In the batch cultivation mode tested in flask, comparable O.D., CDW, and PHA content were achieved by both mono and co-culture systems (data not shown).

Polyhydroxyalkanoate content in the microbial cells was analysed by gas chromatography, GC:
Dry cells were subjected to methanolysis condition in chloroform and acidic methanol (15% H₂SO₄) for 3-4 hours at 100°C. The separated chloroform phase was subjected to GC analysis to quantitatively measure the PHA content against pure PHA standards. GC conditions: The system used was Agilent 7890B, equipped with Zebron ZB-5HT Inferno column (15 m * 0.32 mm * 0.1 um) and FID detector operated at 380°C. Hydrogen was used as the carrier gas. Oven temperature was programmed to increase from 70°C to 350°C (10°C/min).

### Conclusion:

The optimized inoculum preparation helped to prepare active inoculum with similar cell density of both strains. Changing inoculum ratio between the two strains and/or the time of co-culturing (time at which the second or first strain to be added to the production culture) can help to optimize the sugar consumption and to reach higher growth rate and polymer productivity.

### Example 3 - Fed-batch fermentation with mono-culture of Cupriavidus necator DSM 545 for PHB production

### Materials and methods:

The strain used was *C*. *necator* DSM 545. PHA production was conducted in the sterile-in-place fermentor Biostat C 7-L. Cultivation was done in the production medium MSM-2, prepared as described in Example 2. The initial fermentation volume was 4 L. Culture temperature was controlled at 30°C and pH at 6.8-7.5 by NaOH (3M) or HCI (3N). Ammonium hydroxide (NH₄OH, 12.5% v/v) was used for pH control and as nitrogen supply during the 1^{st} stage of the fed-batch fermentation process to support high cell density growth. Nitrogen limited condition was applied by switching ammonium to sodium hydroxide to trigger the PHA biosynthesis stage, after 36-38 hour of inoculation. Dissolved oxygen (dO₂) was controlled at 20% (v/v) of saturation, using compressed air supply 0.5-2.0 (vvm) and stirring 400-900 rpm. The feeding solution was 70% w/v hydrolysed sucrose, prepared as described in Example 1. Nitrogen limited conditions were applied after 24 h of inoculation.

### Results:

The glucose utilizing strain *C*. *necator* DSM 545 was cultivated individually as mono-culture and fed with hydrolysed sucrose solution. In this example, we investigated the reported inability of the glucose utilizing mutant to completely consume both sucrose monosugars, glucose and fructose during fed-batch fermentation which is usually the preferred cultivation mode in PHA industry to reach high-cell density growth and polymer accumulation. The culture was fed with hydrolysed sucrose solution (70% w/v). Figure 1 shows that the strain DSM 545 was able to utilize both sugars, glucose and fructose, at least to some extent during feeding. A relatively high cell density can be reached, 70 g/L and PHB content of 59% w/w. However, during the early stationary phase (which corresponds to the PHA biosynthesis phase), fructose accumulates, meaning that strain DSM 545 prefer glucose over fructose during high cell density growth and polymer biosynthesis phase. The accumulation of fructose happened even though feeding was stopped 2 hours before harvesting (at 51 hour). Similar results have been reported before for this strain when cultivated in presence of both sugars, glucose and fructose (Dalsasso et al. 2019). A proteomic study done by Raberg et al. (2011), has also proven that glucose can support PHA biosynthesis by the mutant strain more than fructose can.

### Conclusion:

*Cupriavidus necator* DSM 545 prefers using glucose over fructose during biosynthesis of PHAs and for high-cell density growth as well.

### Example 4 - Fed-batch fermentation with co-culture of Cupriavidus necator DSM 428 and Cupriavidus necator DSM 545for PHB production from hydrolysed sucrose

### Materials and methods:

The co-culture system for PHB production from hydrolysed sucrose was done by the two strains *C*. *necator* H16 (DSM 428) and *C*. *necator* H1G⁺3 (DSM 545). PHB fermentative production was conducted in the glass fermentor Applikon BioBundle 7-L. Fermentation started with 3L MSM-2 as initial volume, and temperature was controlled at 30°C and pH at 6.8-7.5. Dissolved oxygen (dO₂) was controlled at 20% of saturation, using compressed air supply 0.5-1.0 (vvm) and stirring at 400-900 rpm. At the early exponential phase, the continuous feeding started using hydrolysed sucrose solution (70% w/v), prepared as described in Example 1. Cell growth and PHA synthesis and sugar consumption were analysed as explained in Example 1 and Example 2, respectively).

### Results:

A co-culture of the wild-type *C*. *necator* H16 DSM 428 and its glucose utilizing mutant H1G⁺3 DSM 545 was designed to achieve complete consumption of both sugars, fructose and glucose, while continuing feeding to reach high cell density and polymer content.

The two sugars were consumed differently at the beginning of the fermentation, mainly because of the difference in growth rate between the two strains. The co-culture was nevertheless able to consume both sugars simultaneously by the end of the fermentation. Fructose accumulation was not detected till the end of fermentation, 67 hour (in comparison to Example 3, Figure 1). A slight increase in residual concentration of glucose over fructose was observed during the fermentation (Figure 2), this may be due to the fact that glucose could be utilized by one of the strains only, while both strains were able to utilize fructose from the continuously fed sugar mixture. Higher cell density (CDW) and PHB content was reached by the optimized continuous feeding of hydrolysed sucrose (91 g/L and 78% w/w, respectively).

### Conclusion:

The co-culture system can help to achieve complete consumption of both sugars and enable extended fed-batch fermentation to achieve higher cell density growth and higher polymer productivity.

### Example 5 - Fed-batch fermentation with mono-culture of Cupriavidus necator DSM 545 - PHBV production from hydrolysed sucrose

### Materials and methods:

The mono-culture was operated with strain *C*. *necator* H1G⁺3 DSM 545. Cultivation conditions and feeding regime were conducted as described in Example 4 in the glass fermentor Applikon BioBundle 7-L, however sodium propionate was used as co-substrate for the production of PHA co-polymer, poly(3HB-co-3HV), (PHBV).

Propionate was supplemented to the hydrolysed sucrose feeding solution (280g sodium propionate) at the beginning of PHA biosynthesis phase.

### Results:

Sugar consumption was also investigated during high-cell density fermentation for co-polymer production, PHBV, with the mono-culture, strain DSM 545.

As shown in Figure 3, the mono-culture achieved high-cell density within 48 hour (53 g/L, CDW), after which no increase in cell dry weight could be detected despite prolonged feeding till 68 hour. This limited growth can be due to the accumulation of fructose and/or the inhibitory effect of the co-substrate.

### Example 6 - Fed-batch fermentation with co-culture of Cupriavidus necator H16 DSM 428 and Cupriavidus necator DSM 545 for PHBV production from hydrolysed sucrose

### Materials and methods:

For the production of the co-polymer PHBV in the co-culture system, the two strains C. *necator* DSM 428 and C. *necator* DSM 545 were also cultivated together. Fed-batch fermentation of the co-culture was done in the glass fermentor Applikon BioBundle 7-L. Cultivation conditions and feeding regime were installed as explained in Example 5.

### Results:

In this example, the co-culture system was also investigated for production of the copolymer PHBV in comparison to the mono-culture system in Example 5. The co-culture system achieved a continued increase in cell density during the 66 h long fermentation fed continuously with the hydrolzed sucrose solution supplemented with propionate ( 280 g sodium propionate), as shown in Figure 4 (73 g/L, CDW).

### Conclusion:

The co-culture system also showed better growth over the mono-culture system for the production of PHA copolymer, PHBV.

### General conclusion:

The co-culture system of strain *C*. *necator* DSM 428 and its glucose utilizing UV mutant *C. necator* DSM 545 succeeded to keep similar consumption rate of the two monosugars from hydrolysed sucrose substrate along continued feeding and achieved higher cell density and PHA polymer productivity compare to the mono-culture system of the glucose utilizing strain. The data in Table 1 summarize the improved sugar consumption, CDW and PHA content of all presented examples for PHB and PHBV fermentative production.

**Table 1. Summary of results obtained for mono and co-culture of C. necator DSM 428 and C. necator DSM 545. (mono-c. = monoculture; co-c. = co-culture)**

| Example | Culture type | Time (h) | CDW (g/L) | PHA (g/L) | Residual glucose (g/L) | Residual fructose (g/L) |
|---|---|---|---|---|---|---|
| 3 | mono-c. | 53 | 70 | 40 | 0.93 | 12.67 |
| 4 | co-c. | 67 | 91 | 71 | 4.92 | 0.89 |
| 5 | mono-c. | 68 | 53 | | | |
| 6 | co-c. | 66 | 73 | | | |

### Example 7. Bioextraction of PHA from co-culture cells

### Materials and methods:

The bioextraction of PHA was studied using a portion of wet cells from the PHA co-culture fermentation in Example 4 equivalent to 40-50 g dry weight, autoclaved in 1 L of basal medium ( Ibrahim et al., 2020) (WO 2016/085396) in Applikon BioBundle 3-L. After autoclaving, culture was inoculated with 50 mL of well-grown *B. pumilus* FH9 inoculum (16-24 h). Fermentation was operated at controlled temperature at 37°C. The dissolved oxygen concentration was kept between 5-20% of saturation by suppling air (0.1-0.5 vvm) and controlling agitation (400-800 rpm). Culture pH was controlled at pH 7.0 - 8.0 by the addition of NaOH or HCI (3N). White PHA granules were recovered by centrifugation and water washing (Ibrahim et al., 2020, WO 2016/085396).

### Results:

In this example, a biobased extraction method was used to extract and recover the intracellular PHA granules synthesized by the co-culture system of strains *C*. *necator* DSM 428 and *C*. *necator* DSM 545. The patented process (Ibrahim et al., 2020, WO 2016/085396) was used to hydrolyse the non-PHA biomass of the co-culture cells by the action of growing a lysing bacterial strain, *B. pumilus* FH9. The wet co-culture cells were used as the sole carbon and nitrogen source to induce the bacillus cells to grow and produce its cell-lysing enzymes/factors converting the PHA-biomass into cell hydrolysate rich in protein (SCP) and releasing PHA granules to be easily recovered by centrifugation and water washing steps.

### Conclusion:

Bioextraction as a method for recovery and isolation of PHA from producing microbial cells has been proven to extract PHA granules from the co-culture system of R. *eutropha.* Moreover, hydrolysate rich in protein (single cell protein, SCP) is also produced.

### References

Bernstein, H. C., Paulson, S. D., and Carlson, R. P. 2012. "Synthetic Escherichia coli Consortia Engineered for Syntrophy Demonstrate Enhanced Biomass Productivity." Journal of Biotechnology 157(1): 159-66. http://dx.doi.org/10.1016/jjbiotec.2011.10.001

Dalsasso, R. R. Pavan, F. A., Bordignon, S. E., de Aragão, G. M. F., Poletto, P. 2019. "Polyhydroxybutyrate (PHB) Production by Cupriavidus necator from Sugarcane Vinasse and Molasses as Mixed Substrate." Process Biochemistry 85 (April): 12-18. https://doi.org/10.1016/j.procbio.2019.07.007.

Grage et al. 2009. "Bacterial Polyhydroxyalkanoate Granules: Biogenesis, Structure, and Potential Use as Nano-/Micro-Beads in Biotechnological and Biomedical Applications" Biomacromolecules 10, 660-669

Ibrahim et al., 2020. Process for Extraction of Bioplastic and Production of Monomers from the Bioplastic. WO 2016/085396.

Ibrahim, M.H.A., and Steinbüchel A.. 2009. "Poly(3-Hydroxybutyrate) Production from Glycerol by Zobellella denitrificans MW1 via High-Cell-Density Fed-Batch Fermentation and Simplified Solvent Extraction." Applied and Environmental Microbiology 75(19).

Kim, H.-Y., Park, J.S., Shin, H. D., and Lee Y. H. 1995. "Isolation of Glucose Utilizing Mutant of Alcaligenes eutrophus, Its Substrate Selectivity, Accumulation of Poly-b-Hydroxybutyrate." Journal of Microbiology (Seoul, Korea) 33(1 (March)): 51-58.

Lu, J., Brigham, C. J., Li, S., and Sinskey A. J. 2016. Biotechnology for Biofuel Production and Optimization Ralstonia eutropha H16 as a Platform for the Production of Biofuels, Biodegradable Plastics, and Fine Chemicals from Diverse Carbon Resources. Elsevier B.V. http://dx.doi.org/10.1016/B978-0-444-63475-7.00012-1.

Mozumder, M. S. I., De Wever, H., Volcke, E. I.P. and Garcia-Gonzalez, L. 2014. "A Robust Fed-Batch Feeding Strategy Independent of the Carbon Source for Optimal Polyhydroxybutyrate Production." Process Biochemistry 49(3): 365-73. http://dx.doi.org/10.1016/j.procbio.2013.12.004.

Raberg, M., Peplinski, K., Heiss, S., Ehrenreich, A., Voigt, B., Döring, C., Bömeke, M., Hecker, M., and Steinbüchel A. 2011. "Proteomic and Transcriptomic Elucidation of the Mutant Ralstonia eutropha G+1 with Regard to Glucose Utilization." Applied and Environmental Microbiology 77(6): 2058-70.

Raberg, M., Volodina E., Lin K., and Steinbüchel A.. 2018. "Ralstonia eutropha H16 in Progress: Applications beside PHAs and Establishment as Production Platform by Advanced Genetic Tools." Critical Reviews in Biotechnology 38(4): 494-510. https://doi.org/10.1080/07388551.2017.1369933.

Rebocho, A. T. et al. 2020. "Preparation and Characterization of Films Based on a Natural p(3HB)/mcl-PHA Blend Obtained through the Co-Culture of Cupriavidus necator and Pseudomonas citronellolis in Apple Pulp Waste." Bioengineering 7(2).

Schlegel, H. G., Kaltwasser, H., and Gottschalk, G. 1961. "Ein Submersverfahren Zur Kultur Wasserstoffoxydierender Bakterien: Wachstumsphysiologische Untersuchungen." Archiv für Mikrobiologie 38(3): 209-22.

Schlegel, H. G., and Gottschalk, G. 1965. "[Utilization Of Glucose By A Mutant Of Hydrogenomonas H 16]." Biochemische Zeitschrift 341: 249-59.

Sen, K. Y., Hussin M. H., and Baidurah S. 2019. "Biosynthesis of Poly(3-Hydroxybutyrate) (PHB) by Cupriavidus necator from Various Pretreated Molasses as Carbon Source." Biocatalysis and Agricultural Biotechnology 17(August 2018): 51-59. https://doi.org/10.1016/j.bcab.2018.11.006.

Sichwart, S., Hetzler S., Broker D., and Steinbüchel. A. 2011. "Extension of the Substrate Utilization Range of Ralstonia eutropha Strain H16 by Metabolic Engineering to Include Mannose and Glucose." Applied and Environmental Microbiology 77(4): 1325-34.

Yu, J. 2014. Process for Producing Microbial copolymers from Sucrose-Containing Feedstocks. WO 2013/072723 A1

Tan G.-Y. A., Chen C.-L., Li L., Ge L., Wang L., Razaad I. M. N., Li Y., Zhao L., Mo Y., and Wang J.-Y. 2014. "Start a Research on Biopolymer Polyhydroxyalkanoate (PHA): A Review." Polymers 6(3): 706-54.

Williams and Martin, 2005. "Applications of Polyhydroxyalkanoates (PHA) in Medicine and Pharmacy" Part 4. Polyesters. Wiley-VCH Verlag GmbH & Co. KGaA.

Koller M., 2020. The Handbook of Polyhydroxyalkanoates - Postsynthetic Treatment, Processing and Application' ISBN-10: 0367541076.

## Claims

1. A co-culture system for production of single-cell protein material, pyruvate and/or pyruvate derived products, the co-culture system comprising a microbial component and a substrate,
wherein the microbial component comprises or consists of a first and a second microbial cell,
wherein the first and the second microbial cells are different strains of the species *Cupriavidus necator,*
wherein the substrate comprises at least a first carbon source and a second carbon source, the first carbon source being different from the second carbon source,
wherein the first microbial cell is capable of metabolizing the first carbon source,
wherein the second microbial cell is capable of metabolizing the second carbon source, and
wherein said pyruvate derived products are
growth-associated metabolites selected from alcohols, biofuels and/or amino acids; and/or homo- and co-polymeric polyhydroxyalkanoates, co-polymeric polythioesters, monomeric hydroxyalkanoates and/or cyanophycin.

2. The co-culture system according to claim 1, wherein the single-cell protein material is biomass and/or hydrolysed biomass of *Cupriavidus necator* cells.

3. The co-culture system according to any one of the preceding claims,
wherein the first and the second microbial cells are each a plurality of microbial cells, and
wherein the first and the second microbial cells are polyhydroxyalkanoate (PHA)-producing microbial cells.

4. The co-culture system according to any one of the preceding claims, wherein the first carbon source is fructose and/or wherein the second carbon source is glucose,
wherein the first microbial cell is capable of metabolising fructose, but is not capable of metabolising glucose, and
wherein the second microbial cell is capable of metabolising glucose.

5. The co-culture system according to any one of the preceding claims, wherein the first microbial cell is *Cupriavidus necator* DSM 428, and/or wherein the second microbial cell is a *Cupriavidus necator* strain capable of using a fatty acid as sole carbon source; or wherein the first microbial cell is a *Cupriavidus necator* strain capable of using a fatty acid as sole carbon source and/or wherein the second microbial cell is *Cupriavidus necator* DSM 545.

6. The co-culture system according to any one of the preceding claims, wherein the first microbial cell is *Cupriavidus necator* DSM 428, and/or
wherein the second microbial cell is a *Cupriavidus* necator strain capable of metabolising glucose, such as *Cupriavidus necator* DSM 545.

7. A process for production of single-cell protein material, pyruvate and/or pyruvate derived products, said process comprising:
a) providing a feedstock;
b) providing a microbial component of a co-culture system according to any one of claims 1 to 6; and
c) contacting the feedstock with the microbial component of the co-culture system, thereby fermenting the feedstock to obtain a fermented composition comprising pyruvate and/or pyruvate derived products,
wherein the pyruvate derived products are growth-associated metabolites selected from alcohols, biofuels and/or amino acids; and/or homo- and co-polymeric polyhydroxyalkanoates, co-polymeric polythioesters, monomeric hydroxyalkanoates and/or cyanophycin.

8. The process according to claim 7, wherein the single-cell protein material is biomass and/or hydrolysed biomass of *Cupriavidus necator* cells.

9. The process according to any one of claims 7 to 8, further comprising a pre-treatment step comprising hydrolysing a raw material comprising sucrose to obtain the feedstock wherein the raw material comprises a concentration of sucrose of 40% w/v or above, such as 50% w/v or above, or such as 60% w/v or above, such as 70% w/v or above, such as about 70% w/v.

10. The process according to any one of claims 7 to 9, wherein the feedstock comprises a concentration of glucose of 20% w/v or above, such as 25% w/v or above or such as 30% w/v or above, such as 35% w/v or above, such as about 35% w/v,
wherein the feedstock comprises a concentration of fructose of 20% w/v or above, such as 25% w/v or above or such as 30% w/v or above, such as 35% w/v or above, such as about 35% w/v, and
wherein glucose and fructose represent at least the 60%, such the at least the 70% such as at least the 80%, such as at least the 90%, such as at least the 95%, such as about 100% of the organic carbon in the feedstock.

11. The process according to any one of claims 7 to 10, wherein the glucose concentration in the fermented composition is of 20 g/l or less, such as of 15 g/L or less, such as of 10 g/L or less, such as of 5 g/L or less, such as of about 1 g/L or less, and
wherein the fructose concentration in the fermented composition is of 20 g/l or less, such as of 15 g/L or less, such as of 10 g/L or less, such as of 5 g/L or less, such as of 1 g/L or less.

12. The process according to any one of claims 7 to 11, wherein the temperature of the fermenting step c) is between 25°C and 35°C, such as between 27°C and 33°C, such as between 29°C and 31°C, such as about 30°C, and
wherein pH of the fermenting step c) is between pH 6 and pH 9, such as between pH 6.5 and 8.5, such as between pH 6.7 and 8.2.

13. The process according to any one of claims 7 to 12, wherein the polyhydroxyalkanoates produced have an average molecular weight of at least 300 kDa, such as between 300 kDa and 2000 kDa, such as between 400 kDa and 1500 kDa, such as between 500 kDa and 1000 kDa, such as between 800 kDa and 2000 kDa.

14. The process according to any one of claims 7 to 13, wherein the polyhydroxyalkanoates produced is a homopolymer of poly(3-hydroxybutyrate), or a co-polymer of poly(3-hydroxybutyrate-co-3-hydroxyvalerate)(PHBV) having a content of 3-hydroxyvalerate of between 5 mol% and 40 mol%, such as of between 5 mol% and 35 mol%, such as of between 5 mol% and 20 mol%, such as of between 10 mol% and 20 mol%, such as of between 15 mol% and 20 mol%.

15. The process according to any one of claims 7 to 14, wherein the titer of polyhydroxyalkanoates produced is at least 3 g/L, such as at least 8 g/L, such as at least 10 g/L, 15 g/L, such as at least 20 g/L, such as at least 25 g/L, such as at least 30 g/L, such as at least 50 g/L, such as at least 75 g/L, such as at least 100 g/L, such as at least 125 g/L, such as at least 150 g/L, and
wherein the polyhydroxyalkanoates produced constitutes at least 50% w/w, such as at least 60% w/w, such as at least 70% w/w, such as at least 80% w/w, such as at least 90% w/w of the cell dry weight.

## Patentansprüche

1. Co-Kultursystem zur Produktion von Einzellerproteinmaterial, Pyruvat und/oder von Pyruvat abgeleiteten Produkten, wobei das Co-Kultursystem eine mikrobielle Komponente und ein Substrat umfasst, wobei die mikrobielle Komponente eine erste und eine zweite mikrobielle Zelle umfasst oder aus diesen besteht,
wobei die erste und die zweite mikrobielle Zelle verschiedene Stämme der Art *Cupriavidus necator* sind,
wobei das Substrat mindestens eine erste Kohlenstoffquelle und eine zweite Kohlenstoffquelle umfasst, wobei sich die erste Kohlenstoffquelle von der zweiten Kohlenstoffquelle unterscheidet,
wobei die erste mikrobielle Zelle fähig ist, die erste Kohlenstoffquelle zu metabolisieren,
wobei die zweite mikrobielle Zelle fähig ist, die zweite Kohlenstoffquelle zu metabolisieren, und
wobei die von Pyruvat abgeleiteten Produkte wachstumsassoziierte Metaboliten, die aus Alkoholen, Biokraftstoffen und/oder Aminosäuren ausgewählt sind; und/oder homo- und copolymere Polyhydroxyalkanoate, co-polymere Polythioester, monomere Hydroxyalkanoate und/oder Cyanophycin sind.

2. Co-Kultursystem nach Anspruch 1, wobei das Einzellerproteinmaterial Biomasse und/oder hydrolysierte Biomasse von Zellen von *Cupriavidus necator* ist.

3. Co-Kultursystem nach einem der vorstehenden Ansprüche, wobei die erste und die zweite mikrobielle Zelle jeweils eine Vielzahl mikrobieller Zellen sind, und
wobei die erste und die zweite mikrobielle Zelle Polyhydroxyalkanoat(PHA) produzierende mikrobielle Zellen sind.

4. Co-Kultursystem nach einem der vorstehenden Ansprüche, wobei die erste Kohlenstoffquelle Fructose ist und/oder wobei die zweite Kohlenstoffquelle Glucose ist,
wobei die erste mikrobielle Zelle fähig ist, Fructose zu metabolisieren, aber nicht fähig ist, Glucose zu metabolisieren, und
wobei die zweite mikrobielle Zelle fähig ist, Glucose zu metabolisieren.

5. Co-Kultursystem nach einem der vorstehenden Ansprüche,
wobei die erste mikrobielle Zelle *Cupriavidus necator* DSM 428 ist, und/oder wobei die zweite mikrobielle Zelle ein Stamm von *Cupriavidus necator* ist, der fähig ist, eine Fettsäure als einzige Kohlenstoffquelle zu verwenden; oder
wobei die erste mikrobielle Zelle ein Stamm von *Cupriavidus necator* ist, der fähig ist, eine Fettsäure als einzige Kohlenstoffquelle zu verwenden, und/oder wobei die zweite mikrobielle Zelle *Cupriavidus necator* DSM 545 ist.

6. Co-Kultursystem nach einem der vorstehenden Ansprüche, wobei die erste mikrobielle Zelle *Cupriavidus necator* DSM 428 ist, und/oder
wobei die zweite mikrobielle Zelle ein Stamm von *Cupriavidus necator* ist, der fähig ist, Glucose zu metabolisieren, wie etwa *Cupriavidus necator* DSM 545.

7. Verfahren zur Produktion von Einzellerproteinmaterial, Pyruvat und/oder von Pyruvat abgeleiteten Produkten, wobei das Verfahren umfasst:
a) Bereitstellen eines Ausgangsmaterials;
b) Bereitstellen einer mikrobiellen Komponente eines Co-Kultursystems nach einem der Ansprüche 1 bis 6; und
c) Inkontaktbringen des Ausgangsmaterials mit der mikrobiellen Komponente des Co-Kultursystems, dadurch Fermentieren des Ausgangsmaterials, um eine fermentierte Zusammensetzung zu erhalten, die Pyruvat und/oder von Pyruvat abgeleitete Produkte umfasst,
wobei die von Pyruvat abgeleiteten Produkte wachstumsassoziierte Metaboliten, die aus Alkoholen, Biokraftstoffen und/oder Aminosäuren ausgewählt sind; und/oder homo- und copolymere Polyhydroxyalkanoate, co-polymere Polythioester, monomere Hydroxyalkanoate und/oder Cyanophycin sind.

8. Verfahren nach Anspruch 7, wobei das Einzellerproteinmaterial Biomasse und/oder hydrolysierte Biomasse von Zellen von *Cupriavidus necator* ist.

9. Verfahren nach einem der Ansprüche 7 bis 8, ferner einen Vorbehandlungsschritt umfassend, der Hydrolysieren eines Rohmaterials, das Sucrose umfasst, um das Ausgangsmaterial zu erhalten, umfasst, wobei das Rohmaterial eine Konzentration von Sucrose von 40 % Gewicht pro Volumen (w/v) oder darüber umfasst, wie etwa 50 % Gewicht pro Volumen (w/v) oder darüber, oder wie etwa 60 % Gewicht pro Volumen (w/v) oder darüber, wie etwa 70 % Gewicht pro Volumen (w/v) oder darüber, wie etwa ungefähr 70 % Gewicht pro Volumen (w/v).

10. Verfahren nach einem der Ansprüche 7 bis 9, wobei das Ausgangsmaterial eine Konzentration von Glucose von 20 % Gewicht pro Volumen (w/v) oder darüber umfasst, wie etwa 25 % Gewicht pro Volumen (w/v) oder darüber oder wie etwa 30 % Gewicht pro Volumen (w/v) oder darüber, wie etwa 35 % Gewicht pro Volumen (w/v) oder darüber, wie etwa ungefähr 35 % Gewicht pro Volumen (w/v),
wobei das Ausgangsmaterial eine Konzentration von Fructose von 20 % Gewicht pro Volumen (w/v) oder darüber umfasst, wie etwa 25 % Gewicht pro Volumen (w/v) oder darüber oder wie etwa 30 % Gewicht pro Volumen (w/v) oder darüber, wie etwa 35 % Gewicht pro Volumen (w/v) oder darüber, wie etwa ungefähr 35 % Gewicht pro Volumen (w/v), und
wobei Glucose und Fructose mindestens 60 %, wie mindestens 70 %, wie mindestens 80%, wie mindestens 90 %, wie mindestens 95 %, wie etwa ungefähr 100 % des organischen Kohlenstoffs in dem Ausgangsmaterial darstellen.

11. Verfahren nach einem der Ansprüche 7 bis 10, wobei die Glucosekonzentration in der fermentierten Zusammensetzung 20 g/l oder weniger beträgt, wie etwa 15 g/L oder weniger, wie etwa 10 g/L oder weniger, wie etwa 5 g/L oder weniger, wie etwa ungefähr 1 g/L oder weniger, und
wobei die Fructosekonzentration in der fermentierten Zusammensetzung 20 g/l oder weniger beträgt, wie etwa 15 g/L oder weniger, wie etwa 10 g/L oder weniger, wie etwa 5 g/L oder weniger, wie etwa 1 g/L oder weniger.

12. Verfahren nach einem der Ansprüche 7 bis 11, wobei die Temperatur des Fermentierungsschritts c) zwischen 25 °C und 35 °C liegt, wie etwa zwischen 27 °C und 33 °C, wie etwa zwischen 29 °C und 31 °C, wie etwa ungefähr 30 °C, und
wobei der pH-Wert des Fermentierungsschritts c) zwischen pH 6 und pH 9 liegt, wie etwa zwischen pH 6,5 und 8,5, wie etwa zwischen pH 6,7 und 8,2.

13. Verfahren nach einem der Ansprüche 7 bis 12, wobei die produzierten Polyhydroxyalkanoate eine mittlere Molekülmasse von mindestens 300 kDa aufweisen, wie etwa zwischen 300 kDa und 2000 kDa, wie etwa zwischen 400 kDa und 1500 kDa, wie etwa zwischen 500 kDa und 1000 kDa, wie etwa zwischen 800 kDa und 2000 kDa.

14. Verfahren nach einem der Ansprüche 7 bis 13, wobei die produzierten Polyhydroxyalkanoate ein Homopolymer von Poly(3-hydroxybutyrat) oder ein Copolymer von Poly(3-hydroxybutyrat-co-3-hydroxyvalerat)(PHBV) ist, das einen Gehalt von 3-Hydroxyvalerat von zwischen 5 Mol-% und 40 Mol-% aufweist, wie etwa von zwischen 5 Mol-% und 35 Mol-%, wie etwa von zwischen 5 Mol-% und 20 Mol-%, wie etwa von zwischen 10 Mol-% und 20 Mol-%, wie etwa von zwischen 15 Mol-% und 20 Mol-%.

15. Verfahren nach einem der Ansprüche 7 bis 14, wobei der Titer der produzierten Polyhydroxyalkanoate mindestens 3 g/L beträgt, wie etwa mindestens 8 g/L, wie etwa mindestens 10 g/L, 15 g/L, wie etwa mindestens 20 g/L, wie etwa mindestens 25 g/L, wie etwa mindestens 30 g/L, wie etwa mindestens 50 g/L, wie etwa mindestens 75 g/L, wie etwa mindestens 100 g/L, wie etwa mindestens 125 g/L, wie etwa mindestens 150 g/L, und
wobei die produzierten Polyhydroxyalkanoate mindestens 50 % Gewicht pro Gewicht (w/w), wie etwa mindestens 60 % Gewicht pro Gewicht (w/w), wie etwa mindestens 70 % Gewicht pro Gewicht (w/w), wie etwa mindestens 80 % Gewicht pro Gewicht (w/w), wie etwa mindestens 90 % Gewicht pro Gewicht (w/w) des Zelltrockengewichts ausmachen.

## Revendications

1. Système de co-culture pour la production de matériau protéique unicellulaire, de pyruvate et/ou de produits dérivés de pyruvate, le système de co-culture comprenant un composant microbien et un substrat, dans lequel le composant microbien comprend ou est constitué d'une première et d'une seconde cellule microbienne,
dans lequel la première et la seconde cellules microbiennes sont des souches différentes de l'espèce *Cupriavidus necator,*
dans lequel le substrat comprend au moins une première source de carbone et une seconde source de carbone, la première source de carbone étant différente de la seconde source de carbone, dans lequel la première cellule microbienne est capable de métaboliser la première source de carbone,
dans lequel la seconde cellule microbienne est capable de métaboliser la seconde source de carbone, et
dans lequel lesdits produits dérivés de pyruvate sont des métabolites associés à la croissance choisis parmi les alcools, les biocarburants et/ou les acides aminés ; et/ou
des polyhydroxyalcanoates homo- et co-polymèriques, des polythioesters co-polymèriques, des hydroxyalcanoates monomèriques et/ou de la cyanophycine.

2. Système de co-culture selon la revendication 1, dans lequel le matériau protéique unicellulaire est de la biomasse et/ou de la biomasse hydrolysée de cellules de *Cupriavidus necator.*

3. Système de co-culture selon l'une quelconque des revendications précédentes,
dans lequel la première et la seconde cellules microbiennes sont chacune une pluralité de cellules microbiennes, et dans lequel la première et
la seconde cellules microbiennes sont des cellules microbiennes productrices de polyhydroxyalcanoate (PHA).

4. Système de co-culture selon l'une quelconque des revendications précédentes, dans lequel la première source de carbone est le fructose et/ou dans lequel la seconde source de carbone est le glucose,
dans lequel la première cellule microbienne est capable de métaboliser le fructose, mais n'est pas capable de métaboliser le glucose, et
dans lequel la seconde cellule microbienne est capable de métaboliser le glucose.

5. Système de co-culture selon l'une quelconque des revendications précédentes, dans lequel la première cellule microbienne est *Cupriavidus necator* DSM 428, et/ou dans lequel la seconde cellule microbienne est une souche de *Cupriavidus necator* capable d'utiliser un acide gras comme seule source de carbone ; ou
dans lequel la première cellule microbienne est une souche de *Cupriavidus necator* capable d'utiliser un acide gras comme seule source de carbone et/ou dans lequel la seconde cellule microbienne est *Cupriavidus necator* DSM 545.

6. Système de co-culture selon l'une quelconque des revendications précédentes, dans lequel la première cellule microbienne est *Cupriavidus necator* DSM 428, et/ou dans lequel la seconde cellule microbienne est une souche de *Cupriavidus necator* capable de métaboliser le glucose, telle que *Cupriavidus necator* DSM 545.

7. Procédé de production de matériau protéique unicellulaire, de pyruvate et/ou de produits dérivés de pyruvate, ledit procédé comprenant :
a) la fourniture d'une charge d'alimentation ;
b) la fourniture d'un composant microbien d'un système de co-culture selon l'une quelconque des revendications 1 à 6 ; et
c) la mise en contact de la charge d'alimentation avec le composant microbien du système de co-culture, fermentant ainsi la charge d'alimentation pour obtenir une composition fermentée comprenant du pyruvate et/ou des produits dérivés de pyruvate, dans lequel les produits dérivés de pyruvate sont des métabolites associés à la croissance choisis parmi les alcools, les biocarburants et/ou les acides aminés ; et/ou des polyhydroxyalcanoates homo- et co-polymèriques, des polythioesters co-polymèriques, des hydroxyalcanoates monomèriques et/ou de la cyanophycine.

8. Procédé selon la revendication 7, dans lequel le matériau protéique unicellulaire est de la biomasse et/ou de la biomasse hydrolysée de cellules de *Cupriavidus necator.*

9. Procédé selon l'une quelconque des revendications 7 à 8, comprenant en outre une étape de prétraitement comprenant l'hydrolyse d'une matière première comprenant du saccharose pour obtenir la charge d'alimentation dans lequel la matière première comprend une concentration de saccharose de 40 % p/v ou plus, telle que 50 % p/v ou plus, ou telle que 60 % p/v ou plus, telle que 70 % p/v ou plus, telle qu'environ 70 % p/v.

10. Procédé selon l'une quelconque des revendications 7 à 9, dans lequel la charge d'alimentation comprend une concentration de glucose de 20 % p/v ou plus, telle que 25 % p/v ou plus, ou telle que 30 % p/v ou plus, telle que 35 % p/v ou plus, telle qu'environ 35 % p/v,
dans lequel la charge d'alimentation comprend une concentration de fructose de 20 % p/v ou plus, telle que 25 % p/v ou plus, ou telle que 30 % p/v ou plus, telle que 35 % p/v ou plus, telle qu'environ 35 % p/v, et
dans lequel le glucose et le fructose représentent au moins les 60 %, tel que les au moins 70 % tel qu'au moins les 80 %, tel qu'au moins les 90 %, tel qu'au moins les 95 %, tel qu'environ 100 % du carbone organique dans la charge d'alimentation.

11. Procédé selon l'une quelconque des revendications 7 à 10, dans lequel la concentration en glucose dans la composition fermentée est de 20 g/l ou moins, telle que de 15 g/l ou moins, telle que de 10 g/l ou moins, telle que de 5 g/l ou moins, telle que d'environ 1 g/l ou moins, et
dans lequel la concentration en fructose dans la composition fermentée est de 20 g/l ou moins, telle que de 15 g/l ou moins, telle que de 10 g/l ou moins, telle que de 5 g/l ou moins, telle que de 1 g/l ou moins.

12. Procédé selon l'une quelconque des revendications 7 à 11, dans lequel la température de l'étape de fermentation c) est comprise entre 25 °C et 35 °C, telle qu'entre 27 °C et 33 °C, telle qu'entre 29 °C et 31 °C, telle qu'environ 30°C, et
dans lequel le pH de l'étape de fermentation c) est compris entre pH 6 et pH 9, tel qu'entre pH 6,5 et 8,5, tel qu'entre pH 6,7 et 8,2.

13. Procédé selon l'une quelconque des revendications 7 à 12, dans lequel les polyhydroxyalcanoates produits ont un poids moléculaire moyen d'au moins 300 kDa, tel qu'entre 300 kDa et 2 000 kDa, tel qu'entre 400 kDa et 1 500 kDa, tel qu'entre 500 kDa et 1 000 kDa, tel qu'entre 800 kDa et 2 000 kDa.

14. Procédé selon l'une quelconque des revendications 7 à 13, dans lequel les polyhydroxyalcanoates produits sont un homopolymère de poly(3-hydroxybutyrate), ou un copolymère de poly(3-hydroxybutyrate-co-3-hydroxyvalérate)(PHBV) ayant une teneur en 3-hydroxyvalérate comprise entre 5 % en moles et 40 % en moles, telle que comprise entre 5 % en moles et 35 % en moles, telle que comprise entre 5 % en moles et 20 % en moles, telle que comprise entre 10 % en moles et 20 % en moles, telle que comprise entre 15 % en moles et 20 % en moles.

15. Procédé selon l'une quelconque des revendications 7 à 14, dans lequel le titre de polyhydroxyalcanoates produits est d'au moins 3 g/l, tel qu'au moins 8 g/l, tel qu'au moins 10 g/l, 15 g/l, tel qu'au moins 20 g/l, tel qu'au moins 25 g/l, tel qu'au moins 30 g/l, tel qu'au moins 50 g/l, tel qu'au moins 75 g/l, tel qu'au moins 100 g/l, tel qu'au moins 125 g/l, tel qu'au moins 150 g/l, et
dans lequel les polyhydroxyalcanoates produits constituent au moins 50 % p/p, tel qu'au moins 60 % p/p, tel qu'au moins 70 % p/p, tel qu'au moins 80 % p/p, tel qu'au moins 90 % p/p du poids sec des cellules.
